# EUROPEAN PATENT SPECIFICATION

(11) **EP 2 196 193 B1**
(45) Date of publication and mention of the grant of the patent: **23.09.2015**
(21) Application number: 09251763.0
(22) Date of filing: 08.07.2009
(51) Int. Cl.: A61K 9/06, A61K 47/10, A61K 47/34, A61K 47/42, A61K 9/00

(54) **Hydrogels for use in removing polyps**
Hydrogele zur Verwendung zur Entfernung von Polypen
Hydrogèles pour l'emploi pour éliminer les polypes

(30) Priority: 01.07.2009 US 496060; 08.07.2008 US 78968 P
(43) Date of publication of application: 16.06.2010
(73) Proprietor: Covidien LP, Mansfield, MA 02048 (US)
(72) Inventor: Kennedy, Jack, Guilford, CT 06437 (US); Abuzaina, Ferass, Shelton, CT 06484 (US); Bennett, Steven, Cheshire CT 06410 (GB); Hadba, Ahamad Robert, Wallingford, CT 06492 (GB)
(74) Representative: Soames, Candida Jane

(56) References cited:
- EP-A1- 2 022 410
- WO-A2-2009/007785
- US-A1- 2003 108 511

## Description

### BACKGROUND

The present disclosure relates generally to biocompatible crosslinked polymers, and methods for preparing and using the same. In embodiments, compositions of the present disclosure may be utilized in surgical procedures, including the removal of polyps.

A polyp is generally a growth that projects from a membrane in the body. The shape of a polyp is often described as pedunculated or sessile. Pedunculated polyps grow on stalks, while sessile polyps may have broad bases and a flat appearance. Often, polyps form on mucous membranes such as those lining the colon, bladder, uterus, cervix, vocal cords, and/or nasal passage and protrude into a body cavity. Polyps are problematic in that they may block a passage, and/or may become cancerous. Generally, the larger the polyp, the more likely it is to become cancerous.

Endoscopic polypectomy procedures are effective in removing pedunculated polyps; however, sessile polyps are often problematic. For example, because of their flat, diffuse appearance, sessile polyps may be difficult to snare and excise with electrocautery. To facilitate excision of some polyps, saline may be injected into the submucosa of a polyp to create an artificial cushion that raises the polyp. However, saline has a short residence time in the submucosa: it usually clears within 4 to 5 minutes after injection.

In addition, large polyps are often difficult to remove as a whole, so they are often excised in piecemeal fashion. After the first excision of polyp tissue, injected solution may escape from the submucosa causing the polyp to collapse, thus making it difficult to remove the remaining portions of the polyp. Although saline may be re-injected, it escapes quickly and is not very effective in raising the remaining portions of the polyp.

Attempts to improve submucosa residence time of injection solutions have been reported. For example, solutions of glycerin, dextrose, hyaluronic acid, and hydroxpropyl cellulose have been reported as injection solutions. The most effective of these has been hyaluronic acid. The average residence time of hyaluronic acid solutions in porcine esophagus is reportedly 21.5 minutes. However, these solutions may still leak out of the submucosal layer once the cushion or polyp is breached during the endoscopic dissection or polypectomy.

US 2003/108511 refers to a system for coating a tissue with a hydrogel, comprising a minimally invasive surgical applicator with two nozzles wherein the first chamber is configured to hold a first component polymerizable by a free radical polymerization mechanism and the second chamber is configured to hold a second component that is a free radical initiator.

WO 2009/007785 discloses a composition topically applied to a body cavity or mucosal surfaces (cranial cavity, thoratic cavity, abdominal cavity, vagina, rectum and penile cavities, the urinary tract, nasal cavity, mouth, eye, ear, peritoneum, large and small bowel, the caecum, bladder, stomach, the cavity between the uterus and the fallopian tubes, ovaries, e.g. in cases of polyps of the colon or the rectum).

In EP 2 022 410 cannulas are utilized to introduce thermally responsive polymers into polyps to aid in their removal.

Thus, there remains room for improvement in compositions and methods for performing endoscopic polypectomy procedures, and especially to minimize, reduce, and/or eliminate the need for multiple polyp injections during excision.

### SUMMARY

The present disclosure provides compositions for use in surgical procedures, including for use in a method for the removal of polyps. In embodiments, a method of the present disclosure may include introducing into tissue, including the submucosa of a polyp, a composition including a biocompatible small molecule crosslinker with a molecular weight of 2000 or less, the crosslinker having n crosslinker functional groups, wherein n is two or more, and wherein the crosslinker functional groups are either electrophilic or nucleophilic. The crosslinker may be administered in combination with a synthetic biocompatible functional polymer with a molecular weight of at least about 7 times more than the crosslinker, the functional polymer having m functional polymer functional groups, wherein m is two or more and the sum of n and m is five or more, and wherein the functional polymer functional groups are nucleophilic if the crosslinker functional groups are electrophilic, and the functional polymer functional groups are electrophilic if the crosslinker functional groups are nucleophilic. The crosslinker and functional polymer may be permitted to react in the submucosa of the polyp to form a hydrogel and the polyp may be removed.

In other embodiments, a use in a method of the present disclosure includes introducing into tissue such as the submucosa of a polyp a composition including a biocompatible small molecule crosslinker having at least two first functional groups and a molecular weight of 2000 or less, with a synthetic biocompatible functional polymer having at least two second functional groups and having a molecular weight at least about 7 times more than the small molecule crosslinker, wherein each of the first functional groups are different than each of the second functional groups, and the first and the second functional groups are electrophiles and nucleophiles. The first functional groups of the crosslinker and the second functional groups of the functional polymer may be permitted to react in the submucosa of the polyp to form a biocompatible crosslinked polymer hydrogel and the polyp may be removed.

In yet other embodiments, a use in a method of the present disclosure may include introducing into tissue including the submucosa of a polyp a composition including at least one biocompatible crosslinker region including a crosslinked synthetic crosslinker molecule with a pre-crosslinked molecular weight of less than 2000, in combination with at least one biocompatible functional polymer region such as a crosslinked synthetic polymer molecule with a pre-crosslinked molecular weight of more than about 7 times the molecular weight of the pre-crosslinked crosslinker molecule. The crosslinker and the functional polymer may be permitted to react in the submucosa of the polyp to form a biocompatible crosslinked polymer hydrogel, wherein the biocompatible crosslinked polymer hydrogel possesses at least three links between the crosslinker region and the functional polymer region, and wherein the links are a reaction product of at least one electrophilic functional group with at least one nucleophilic functional group. Once the hydrogel has formed, the polyp may be removed.

The invention may be described by reference to the following numbered paragraphs:

A composition comprising a biocompatible small molecule crosslinker with a molecular weight of 2000 or less, the crosslinker having n crosslinker functional groups, wherein n is two or more, and wherein the crosslinker functional groups are either electrophilic or nucleophilic, in combination with a synthetic biocompatible functional polymer with a molecular weight of at least about 7 times more than the crosslinker, the functional polymer having m functional polymer functional groups, wherein m is two or more and the sum of n and m is five or more, and wherein the functional polymer functional groups are nucleophilic if the crosslinker functional groups are electrophilic, and the functional polymer functional groups are electrophilic if the crosslinker functional groups are nucleophilic for use in a method wherein the method comprises introducing into tissue comprising submucosa of a polyp said composition in combination with said synthetic biocompatible functional polymer and permitting the crosslinker and functional polymer to react in the submucosa of the polyp to form a hydrogel; and
removing the polyp.

The composition of paragraph [0012], wherein providing a biocompatible small molecule crosslinker further comprises providing a biocompatible small molecule crosslinker having a solubility of at least 1 g/100 ml in an aqueous solution; or the composition of paragraph [0012], wherein providing a biocompatible small molecule crosslinker further comprises providing a biocompatible small molecule crosslinker having crosslinker functional groups that are electrophilic; preferably wherein providing a biocompatible small molecule crosslinker having crosslinker functional groups that are electrophilic further comprises providing a biocompatible small molecule crosslinker wherein the electrophilic crosslinker functional groups are N-hydroxysuccinimide-based crosslinker groups; or wherein providing a synthetic biocompatible functional polymer further comprises providing a synthetic biocompatible functional polymer wherein the functional polymer functional groups are amines.

The composition of paragraph [0012], wherein providing a biocompatible small molecule crosslinker further comprises providing a biocompatible small molecule crosslinker having crosslinker functional groups that are nucleophilic; preferably wherein providing a biocompatible small molecule crosslinker having crosslinker functional groups that are nucleophilic further comprises providing a biocompatible small molecule crosslinker wherein the crosslinker functional groups are amines or wherein providing a synthetic biocompatible functional polymer further comprises providing a synthetic biocompatible functional polymer wherein the functional polymer functional groups are N-hydroxysuccinimide groups.

The composition of paragraph [0012], wherein providing a biocompatible small molecule crosslinker further comprises providing a biocompatible small molecule crosslinker having a biodegradable link.

The composition of paragraph [0012], wherein providing a synthetic biocompatible functional polymer further comprises providing a synthetic biocompatible functional polymer having a biodegradable link; or the composition of paragraph [0012], wherein permitting the crosslinker and functional polymer to react further comprises reacting the crosslinker functional groups and the functional polymer functional groups to produce a biodegradable link; or the composition of paragraph [0012], wherein the hydrogel further comprises a dye; or the composition of paragraph [0012], wherein the hydrogel further comprises one or more active ingredients; preferably wherein the active ingredient comprises enzymes, vasoconstrictors, chemotherapeutic agents, antimicrobials, antibiotics, and combinations thereof.

The composition of paragraph [0012], wherein the hydrogel forms over a period of time of from about 5 seconds to about 90 seconds.

A composition comprising a biocompatible small molecule crosslinker having at least two first functional groups and a molecular weight of 2000 or less with a synthetic biocompatible functional polymer having at least two second functional groups and having a molecular weight at least about 7 times more than the small molecule crosslinker, wherein each of the first functional groups are different than each of the second functional groups and the first and the second functional groups are chosen from the group consisting of electrophiles and nucleophiles; for use in a method wherein the method comprises the steps of introducing into tissue comprising submucosa of a polyp said composition and permitting the first functional groups of the crosslinker and the second functional groups of the functional polymer to react in the submucosa of the polyp to form a biocompatible crosslinked polymer hydrogel; and
removing the polyp.

The composition of paragraph [0018], wherein the small molecule crosslinker is selected from the group consisting of dilysine, trilysine, tetralysine, and Tris; or the composition of paragraph [0018], wherein the small molecule crosslinker is selected from the group consisting of ornithine, spermine, spermidine, urea, guanidine, diamniopimelic acid, diaminobutyric acid, methylornithine, diaminopropionic acid, cystine, lanthionine, cystamine, trioxatridecanediamine, cyclohexanebis(methylamine), tetraethylenepentamine, pentaethylenehexamine, methylenebis(methylcyclohexamine), diaminocyclohexane, n-(2-aminoethyl)-1,3-propanediamine, diaminomethyldipropylamine, iminobispropylamine, bis(hexamethlyene)triamine, triethylenetetramine, bis(aminopropyl)ethylenediamine, bis(2-aminoethyl)-1,3-propanediamine, bis(aminopropyl)propanediamine, diamniomethylpropane, 1,2-diamino-2-methylpropane, 1,3-diaminopentane, dimethylpropanediamine, 2,2-dimethyl 1,3-propanediamine, methylpentanediamine, 2-methyl-1,5 pentanediamine, diaminoheptane, diaminooctane, diaminononane, diaminodecane, and diaminododecane; or the composition of paragraph [0018], wherein the formation of the biocompatible crosslinked polymer requires less than about 45 seconds as measured by a gel time measurement; or the composition of paragraph [0018] wherein the small molecule crosslinker has at least 3 functional groups; or the composition of paragraph [0018], wherein the concentration of solids in the biocompatible crosslinked polymer hydrogel is from about 8 percent by weight to about 20 percent by weight; or the composition of paragraph [0018], wherein the first functional groups comprise amines and the second functional groups comprise succinimides; or the composition of paragraph [0018], wherein the hydrogel forms over a period of time of from about 5 seconds to about 90 seconds.

A composition comprising at least one biocompatible crosslinker region comprising a crosslinked synthetic crosslinker molecule with a pre-crosslinked molecular weight of less than 2000, in combination with at least one biocompatible functional polymer region consisting essentially of a crosslinked synthetic polymer molecule with a pre-crosslinked molecular weight of more than about 7 times the molecular weight of the pre-crosslinked crosslinker molecule; for use in a method wherein the method comprises the steps of introducing into tissue comprising submucosa of a polyp said composition in combination with said at least one biocompatible functional polymer region; permitting the crosslinker and the functional polymer to react in the submucosa of the polyp to form a biocompatible crosslinked polymer hydrogel wherein the biocompatible crosslinked polymer hydrogel comprises at least three links between the crosslinker region and the functional polymer region, and wherein the links are a reaction product of at least one electrophilic functional group with at least one nucleophilic functional group; and
removing the polyp.

The composition of paragraph [0020], wherein the biocompatible crosslinker region has a solubility of at least 1 g/100 ml in an aqueous solution; or the composition of paragraph [0020] wherein the biocompatible crosslinked polymer hydrogel further comprises at least one biodegradable link; or the composition of paragraph [0020], wherein at least one of the links between the crosslinker and functional polymer region is biodegradable; or the composition of paragraph [0020], wherein the crosslinker is selected from the group consisting of dilysine, trilysine, tetralysine, and Tris.

The composition of paragraph [0020], wherein the crosslinker is selected from the group consisting of ornithine, spermine, spermidine, urea, guanidine, diamniopimelic acid, diaminobutyric acid, methylornithine, diaminopropionic acid, cystine, lanthionine, cystamine, trioxatridecanediamine, cyclohexanebis(methylamine), tetraethylenepentamine, pentaethylenehexamine, methylenebis(methylcyclohexamine), diaminocyclohexane, n-(2-aminoethyl)-1,3-propanediamine, diaminomethyldipropylamine, iminobispropylamine, bis(hexamethlyene)triamine, triethylenetetramine, bis(aminopropyl)ethylenediamine, bis(2-aminoethyl)-1,3-propanediamine, bis(aminopropyl)propanediamine, diamniomethylpropane, 1,2-diamino-2-methylpropane, 1,3-diaminopentane, dimethylpropanediamine, 2,2-dimethyl 1,3-propanediamine, methylpentanediamine, 2-methyl-1,5 pentanediamine, diaminoheptane, diaminooctane, diaminononane, diaminodecane, and diaminododecane.

The composition of paragraph [0020], wherein the hydrogel forms over a period of time of from about 5 seconds to about 90 seconds.

### BRIEF DESCRIPTION OF THE DRAWINGS

FIGS. 1A-1E depicts electrophilic functional group water soluble and biodegradable crosslinkers or functional polymers, which can be crosslinked with appropriate nucleophilic functional group precursors.

FIGS. 2F-2J depicts nucleophilic water soluble and biodegradable crosslinkers or functional polymers, which can be crosslinked with appropriate electrophilic precursors.

FIGS. 3K-3O depicts electrophilic water soluble and biodegradable crosslinkers or functional polymers, which can be crosslinked with appropriate nucleophilic functional group precursors, wherein either the biodegradable linkages or the functional groups are selected so as to make the precursor water soluble.

FIGS. 4P-4T depicts nucleophilic functional group water soluble crosslinkers or functional polymers, which can be crosslinked with appropriate electrophilic functional group precursors, and which are not biodegradable.

FIGS. 5U-5X depicts electrophilic water soluble crosslinkers or functional polymers, which can be crosslinked with appropriate nucleophilic functional group precursors, and which are not biodegradable.

FIG. 6 depicts certain N-hydroxysuccinimide (NHS) esters.

FIG. 7 shows the variation in gelation time plotted against the number of amino groups for the precursors of Examples 2-4, wherein 4 arm 10 kDa succinimidyl glutarate PEG ("SG-PEG") was reacted with di-, tri- or tetra-lysine.

FIG. 8 shows variation in gelation time with the solution age of the electrophilic functional polymers of Examples 2-4, see also Example 6.

FIG. 9 shows the variation in gelation time with the concentration of biocompatible crosslinked polymer precursors, and with the solution age of the 4 arm 10 kDa carboxymethyl - hydroxybutyrate -N- hydroxysuccinimidyl PEG ("CM-HBA-NHS") electrophilic functional polymer of Example 7.

FIG. 10 shows variation in degradation time with concentration for the biocompatible crosslinked polymer of Example 8.

FIG. 11 depicts the chemical structure of trilysine (LLL).

FIG. 12 depicts the chemical structure of Tris.

FIG 13 is a graph of an example of a gel time of a hydrogel as a function of Tris content in an LLL-Tris hydrogel, as is further described in Example 13.

FIG. 14 is a graph of swelling of a hydrogel as a function of Tris content in an LLL-Tris hydrogel, as is further described in Example 13.

FIG 15 is a graph of degradation of a hydrogel as a function of Tris content in an LLL-Tris hydrogel, as is further described in Example 13.

FIG 16 is a graph of a modulus of a hydrogel as a function of Tris content in an LLL-Tris hydrogel, as is further described in Example 13.

FIG 17 is a graph of degradation of a Tris-based hydrogel, as is further described in Example 15.

FIG 18 is a graph of degradation of an LLL-based hydrogel, as is further described in Example 15.

FIG 19 depicts a four-armed CM-HBA-NHS, an electrophile made with polyethylene glycol and NHS esters.

FIG 20 depicts 4a PEG-SG-NHS, a four armed succinimidyl glutarate made with polyethylene glycol and NHS.

FIG 21 depicts the degradation of hydrogels formed with dilysine (LL or Lys-Lys), see also Example 16.

FIG 22 depicts the gel time of hydrogels formed with dilysine (LL or Lys-Lys) as a function of solids concentration, see also Example 16.

FIG 23 depicts the swelling of hydrogels formed with dilysine (LL or Lys-Lys) as a function of solids concentration, see also Example 16.

FIG 24 depicts the effect of electrophile pot life on gel time for hydrogels made with dilysine (LL or Lys-Lys), see also Example 16.

FIG. 25 depicts a chemical structure for spermidine.

FIG. 26 depicts a chemical structure for spermine.

FIG. 27 depicts a chemical structure for ornithine.

FIG 28 depicts a chemical structure for dilysine (LL, or Lys-Lys).

FIG 29 is a bar graph of gelation time as a function of the number of reactive amines per nucleophile, see also Example 17.

FIG 30 is a bar graph of swelling as a function of the number of reactive amines per nucleophile, see also Example 17.

FIG 31 is a bar graph of degradation time as a function of the number of reactive amines per nucleophile, see also Example 17.

FIG 32 is a bar graph of gelation time as a function of pH, see also Example 17.

FIG. 33 is a bar graph of swelling as a function of pH, see also Example 17.

FIG. 34 is a bar graph of degradation as a function of pH, see also Example 17.

FIG. 35 is a bar graph of gel time as a function of solids and electrophilic pot life, see also Example 17.

FIG. 36 is a bar graph of swelling as a function of solids content, see also Example 17.

FIG. 37 is a bar graph of degradation as a function of solids and electrophilic pot life, see also Example 17.

FIG. 38 depicts the chemical structure of JEFFAMINE®.

FIG. 39 is a bar graph of the gel time for hydrogels made with LUPASOL®, Tris, LLL, or spermidine, plotted as a function of amine content, see Example 18.

FIG 40 is a bar graph of the swelling for various hydrogels, see Example 18.

FIG. 41 is a bar graph of the degradation time for various hydrogels, see Example 18.

FIG. 42 shows the relationship between gel time and pot life as a function of solids content, pH, and stoichiometry of electrophiles: amines, see Example 19.

FIG. 43 shows the relationship between swelling and solids content, pH, and stoichiometry of electrophiles: amines, see Example 19.

FIG. 44 shows the relationship between degradation and solids content, pH, and stoichiometry of electrophiles:amines, see Example 19.

FIG. 45 shows the relationship between modulus and solids content, pH, and stoichiometry of electrophiles:amines, see Example 19.

FIG. 46 is a graph showing the degradability of hydrogels made from multiarmed electrophiles mixed with low molecular weight amines.

### DETAILED DESCRIPTION

The degradation rate of a hydrogel implant in a patient is an important aspect of the hydrogel. It may be desirable for many applications to make a hydrogel that is readily degradable. Further, it may be desirable for many applications that the hydrogel be formulated so that its degradability is predictable. The present disclosure provides embodiments directed to readily degradable hydrogels that have a predictable degradation profile. These readily degradable hydrogels may have multiple uses. In embodiments they may be introduced into a polyp or the submucosa of a polyp and utilized to facilitate removal of the polyp during a polypectomy procedure.

Previously, biodegradable implants were made of polylactides and/or polyglycolides, or other polymers having contiguous esters. The esters degrade in water, a process termed hydrolysis. Polyglycolides are, in general, absorbed by a patient's body in the space of a few months, whereas polylactides require longer times. Polylactides include Poly-L-lactide, which is generally crystalline in structure, and Poly-DL-lactide, which is generally amorphous. The crystalline structure resorbs more slowly because it is hydrated more slowly. Since these polymers, generally speaking, degrade in a few months' time span, it is difficult to make readily degradable implants using these materials.

One would normally predict that a polyester would degrade more rapidly than an isolated ester because the average time for hydrolysis of the first ester in a group of esters should be shorter than the time required for just one ester. In other words, if an ester were to require one to three days to hydrolyze in water, then it is more likely that one out of ten esters would hydrolyze before one out of one esters would hydrolyze. Therefore, it would normally be expected that a polymer having multiple adjacent esters would degrade more rapidly than polymers having the same number of isolated esters, or a polymer having fewer isolated esters. It is unexpected, therefore, that hydrogels made using isolated esters would readily degrade under circumstances where hydrogels made of multiple adjacent esters do not readily degrade.

Certain embodiments set forth herein, however, include hydrogels made from combinations of polymers having isolated esters plus low molecular weight amines that degrade readily. The term "isolated ester" as used herein is an ester that is not adjacent to another ester. The term "low molecular weight amine" as used herein is a molecule having at least two primary amine groups and a molecular weight of less than about 1000. Without being bound to any particular theory, it is believed that unreacted primary amines present in the hydrogel enhance the basicity of water in the region next to the isolated esters, so that their hydrolysis is accelerated. Further, or alternatively, tension in the polymers created by the short length of the low molecular weight amine may serve to accelerate degradation.

Various examples of the use of gels or hydrogels in the body, or for various other uses, are described in, for example, U.S. Patent Nos. 6,020,326, 5,874,500, 5,814,621, 5,605,938, 5,527,856, 5,550,188, 4,414,976, 4,427,651, and 4,925,677; the entire disclosures of each of which are incorporated by reference herein, to the extent they do not contradict what is explicitly disclosed herein.

In accordance with the present disclosure, isolated esters may be utilized in forming the hydrogel which may be readily degradable. Readily degradable means being approximately completely degraded after less than approximately six months, as indicated by an essentially complete loss of mechanical strength. Further, the use of low molecular weight amines in certain compositions and methods has yielded unexpected results that have not been previously expected or appreciated.

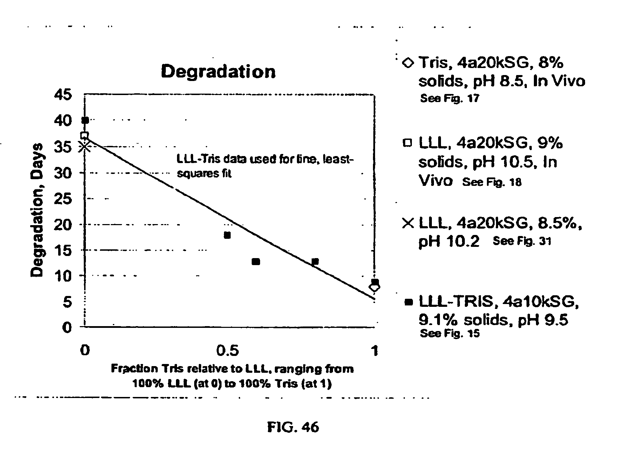

One aspect of the use of combinations of low molecular weight amines is that they unexpectedly have an approximately linear degradation profile. Referring to Figure 46, the degradation time for hydrogels made of mixtures of low molecular weight amines, for example Tris and trilysine, is approximately linearly dependent upon the relative amounts of the low molecular weight amines. A predictable degradation rate may be useful for designing devices and materials that require degradation within a certain time window.

In accordance with the present disclosure, isolated esters may be utilized for making readily degradable materials. Multiple low molecular weight amines may also be utilized to make readily degradable hydrogels. An in vitro gel time disappearance test may be used to approximate in vivo degradability, as described in Example 13, which sets forth procedures that a person of ordinary skill in these arts may use to determine degradation. Embodiments of readily degradable gels that degrade in less than six months are contemplated, including gel degradation times of from about one day to about six months, and any time in between one day and six months.

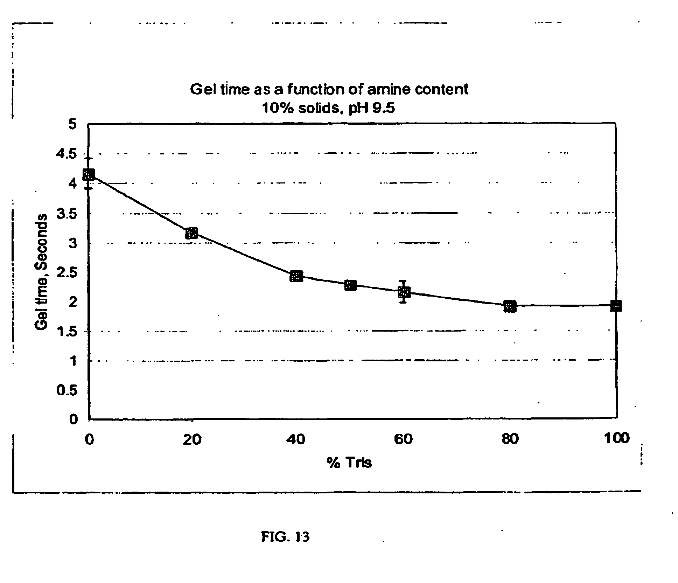

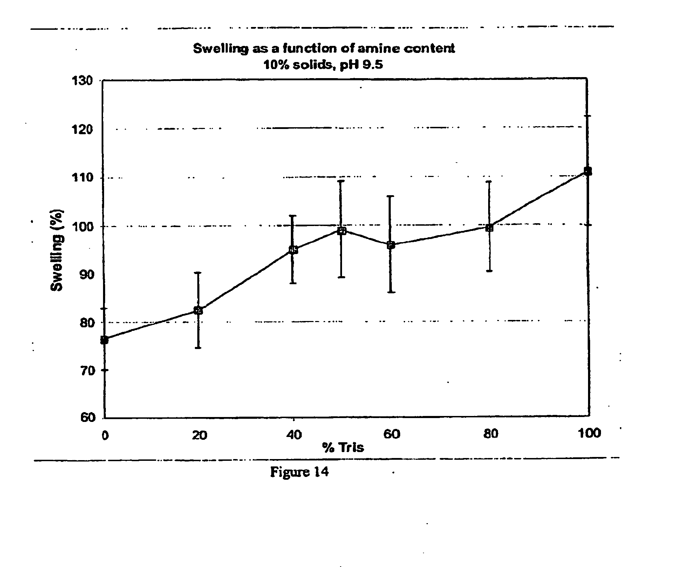

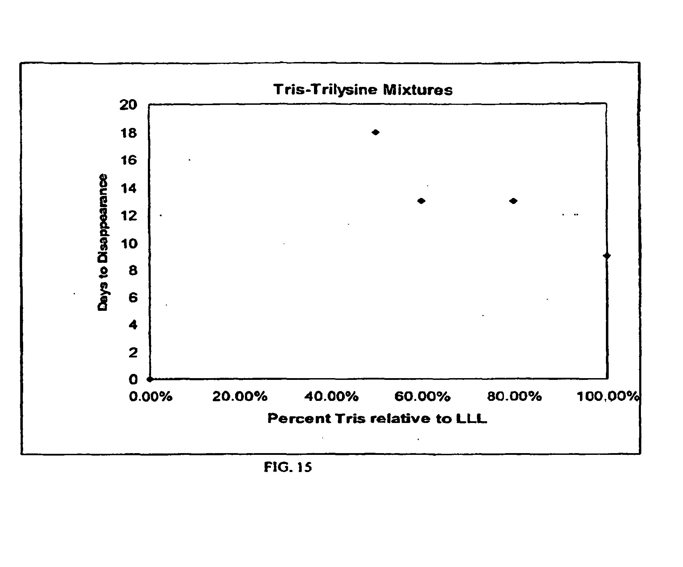

The combination of at least two low molecular weight amines with a polymer having at least one isolated ester to make a hydrogel having isolated esters may be particularly effective in making a degradable gel. For instance, Example 13 shows that the combination of Tris and trilysine accelerates gelation and degradation rates compared to formulations possessing only trilysine (Figures 13-15).

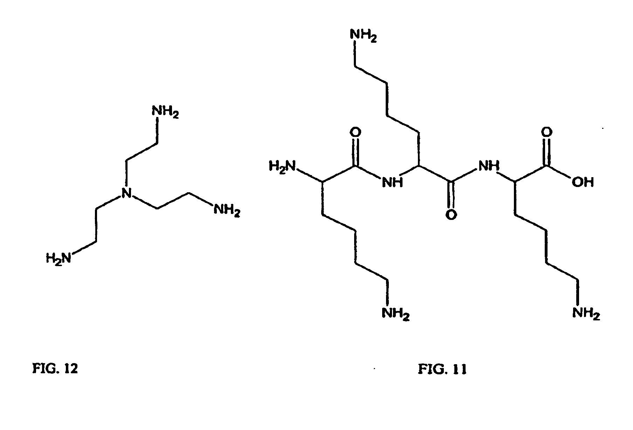

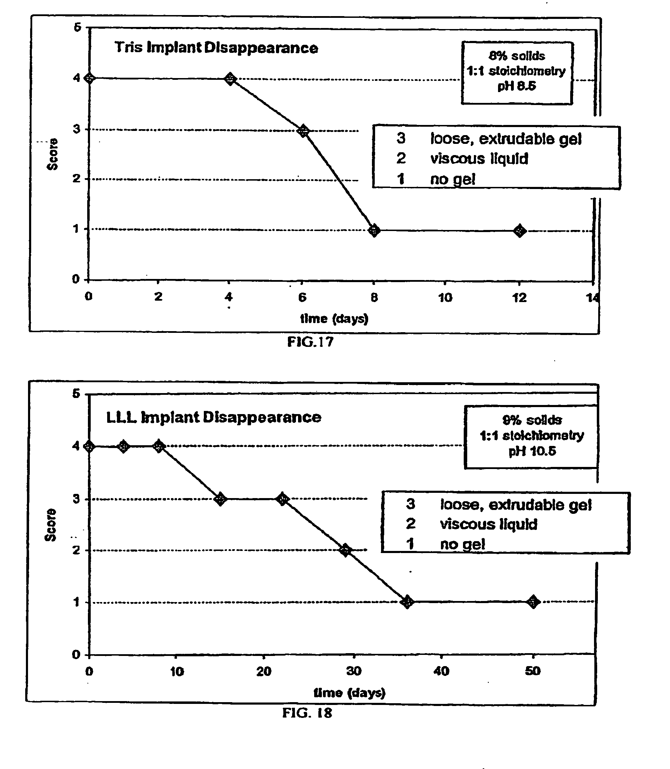

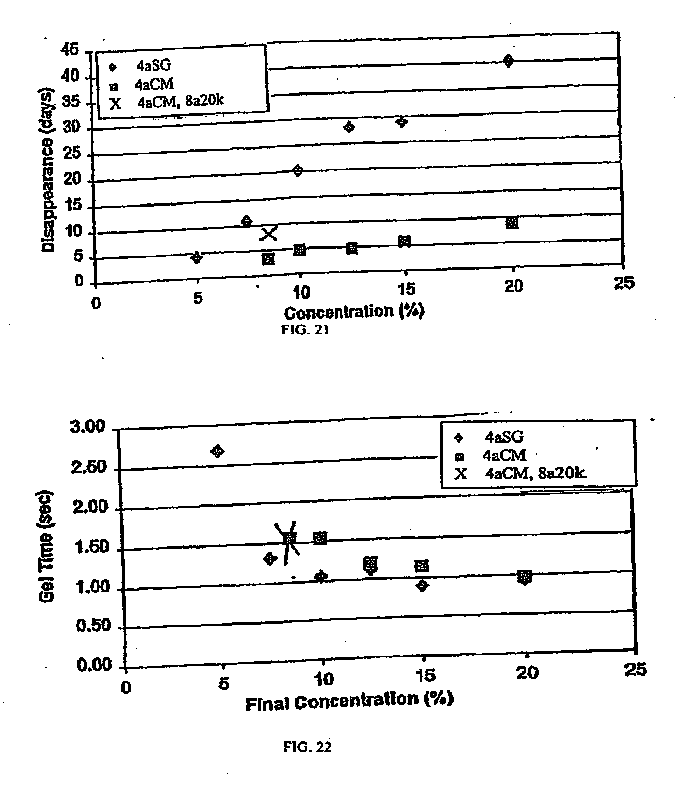

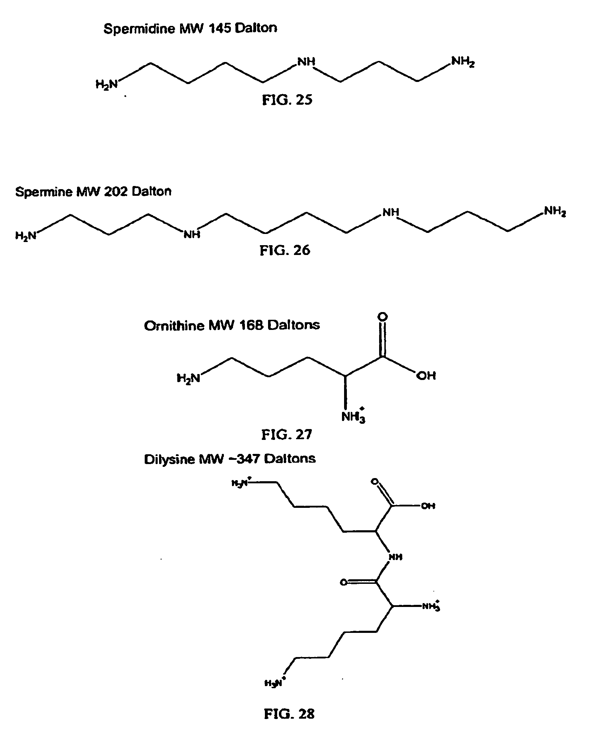

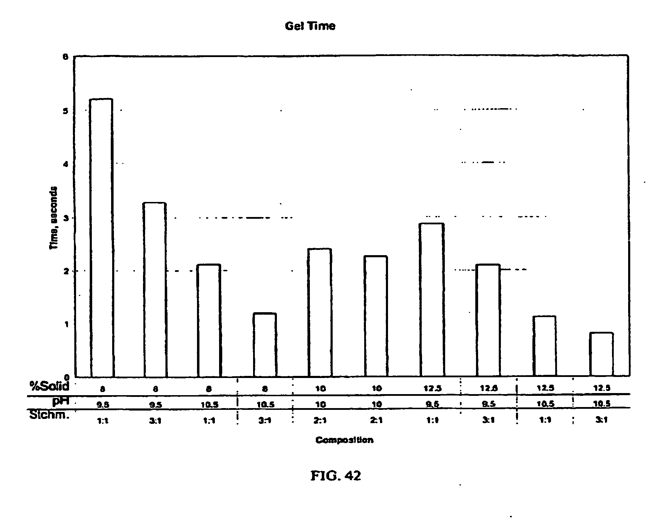

Detailed methods and procedures for using low molecular weight amines in conjunction with isolated ester precursors are set forth herein. For example, Figures 17 and 18 (Examples 14 and 15) show the persistence of isolated ester hydrogels made using the low molecular weight amine trilysine, or the combination of Tris with trilysine, in vivo. The properties of isolated ester hydrogels made using dilysine were also examined, as shown in Figures 21-23 (Example 16). Multiple low molecular weight amines were used in Examples 17 and 18 to make hydrogels that contained isolated esters (Figures 11, 12, 25-39). Aspects relating to the solids content, pH, pot life, swelling, degradation, gel time, and modulus are set forth in the Examples. Example 19 provides a detailed example of maintaining hydrogels having a predictable degradability using combinations of low molecular weight amines in conjunction with isolated esters (Figures 42-45). Examples 20 and 21 set forth a detailed study involving the low molecular weight amine dilysine in conjunction with various electrophiles having isolated esters.

An embodiment of the present disclosure involves a mixture or a process of mixing hydrophilic reactive precursor species having nucleophilic functional groups with hydrophilic reactive precursor species having electrophilic functional groups such that they form a mixture that crosslinks quickly after contact with the tissue of a patient to form a biodegradable hydrogel that coats and adheres to a tissue. This may be achieved by making reactive precursor species that crosslink quickly after mixing. Hydrophilic reactive precursor species can be dissolved in buffered water such that they provide low viscosity solutions that readily mix and flow when contacting the tissue. As they flow across the tissue, they conform to the shape of the small features of the tissue such as bumps, crevices and any deviation from molecular smoothness. If the reactive precursor species are too slow to crosslink, they will flow off the tissue and away into other portions of the body with the result that the user will be unable to localize the hydrogel on the desired tissue. Without limiting the compositions of the present disclosure to a particular theory of operation, it is believed that reactive precursor species that crosslink quickly after contacting a tissue surface will form a three dimensional structure that is mechanically interlocked with the coated tissue. This interlocking contributes to adherence, intimate contact, and essentially continuous coverage of the coated region of the tissue.

Adherence is important for medical applications that require a coating, e.g., for prevention of adhesions, since a user must be able to place the hydrogel in the portions of the patient that are needful, for example, around an ovary or surrounding an intestine. Adherence is also important for placement of the hydrogel in a polyp or the submucosa adjacent a polyp, to minimize leakage after the first portion of a polyp has been removed. Further, the hydrogel should remain on or in the intended tissue. The hydrogels have good adhesion to tissue and are useful for all applications wherein surgical glues or adhesives have previously been used. For example, introduction into a polyp to facilitate removal of the polyp during a polypectomy may be accomplished with combinations of reactive precursor species described herein by using reactive precursor species with nucleophilic functional groups for mixing with hydrophilic reactive precursor species having electrophilic functional groups to form a mix that crosslinks quickly after contact with the tissue of a patient, e.g., a polyp or the submucosa of a polyp, to form a hydrogel that raises a polyp from the submucosa surface.

Suitable crosslinking times vary for different applications. In most applications, the crosslinking reaction leading to gelation may occur within about 10 minutes, in embodiments within about 2 minutes, in other embodiments within about 10 seconds. Methods for introducing the hydrogels and/or its components in vivo include, for example, those disclosed in U.S. Patents 6,179,862; 6,165,201; 6,152,943; and 6,610,033, the entire disclosures of each of which are incorporated by reference herein, to the extent they do not contradict what is explicitly disclosed. In the case of introduction into a polyp or the submucosa adjacent the polyp, a suitable time for gelation may be from about 5 seconds to about 90 seconds, in embodiments from about 10 seconds to about 30 seconds.

### Functional Groups

Each precursor may be multifunctional, meaning that it includes two or more electrophilic or nucleophilic functional groups, such that a nucleophilic functional group on one precursor may react with an electrophilic functional group on another precursor to form a covalent bond. At least one of the precursors includes more than two functional groups, so that, as a result of electrophilic-nucleophilic reactions, the precursors combine to form crosslinked polymeric products. Such reactions may be referred to as "crosslinking reactions".

In embodiments, each precursor includes only nucleophilic or only electrophilic functional groups, so long as both nucleophilic and electrophilic precursors are used in the crosslinking reaction. Thus, for example, if a crosslinker has nucleophilic functional groups such as amines, the functional polymer may have electrophilic functional groups such as N-hydroxysuccinimides. On the other hand, if a crosslinker has electrophilic functional groups such as N-hydroxysuccinimides or sulfosuccinimides, then the functional polymer may have nucleophilic functional groups such as amines or thiols. Thus, functional polymers such as proteins, poly(allyl amine), or amine-terminated di-or multifunctional poly(ethylene glycol) ("PEG") can be used.

### Low Molecular Weight Amine Precursors and Hydrogels

Some embodiments include the use of low molecular weight amines as the crosslinker. Such low molecular weight amines may have at least two primary amines and a molecular weight of less than about 1000. Examples of such low molecular weight amines include dilysine, trilysine, tetralysine, and Tris. Following the nomenclature set forth in the Aldrich Catalog of 2002, other such examples include ornithine, spermine, spermidine, urea, guanidine, diamniopimelic acid, diaminobutyric acid, methylornithine, diaminopropionic acid, cystine, lanthionine, cystamine, trioxatridecanediamine, cyclohexanebis(methylamine), tetraethylenepentamine, pentaethylenehexamine, methylenebis(methylcyclohexamine), diaminocyclohexane, n-(2-aminoethyl)-1,3-propanediamine, diaminomethyldipropylamine, iminobispropylamine, bis(hexamethlyene)triamine, triethylenetetramine, bis(aminopropyl)ethylenediamine, bis(2-aminoethyl)-1,3-propanediamine, bis(aminopropyl)propanediamine, diamniomethylpropane, 1,2-diamino-2-methylpropane, 1,3-diaminopentane, dimethylpropanediamine, 2,2-dimethyl 1,3-propanediamine, methylpentanediamine, 2-methyl-1,5 pentanediamine, diaminoheptane, diaminooctane, diaminononane, diaminodecane, diaminododecane, and the like.

Low molecular weight amines may be used to make hydrogels by combining them with suitable electrophiles such as those set forth herein. A low molecular weight amine having only two primary amines would normally require combination with an electrophile having at least three arms in order to achieve a cross-linked hydrogel. The low molecular weight amines may be used in combination with other nucleophiles set forth herein, including multiarmed polyethylene glycols. For example, Tris may be combined with, dilysine, trilysine, tetralysine, or other low molecular weight amines. Combinations of at least one low molecular weight amine are contemplated, including combinations of two, three, four, or more. Combinations of low molecular weight amines with proteins, degradable polymers, and other materials as set forth herein are also contemplated.

Examples of how to produce such gels include those disclosed in, for example, U.S. Patent Nos. 7,347,850, 7,332,566, 7,009,034, and 6,566,406, the entire disclosures of each of which are incorporated by reference herein.

### Water Soluble Cores

The precursors may have biologically inert and water soluble cores. When the core is a polymeric region that is water soluble, suitable polymers that may be used include: polyethers, for example, polyalkylene oxides such as polyethylene glycol("PEG"), polyethylene oxide ("PEO"), polyethylene oxide-co-polypropylene oxide ("PPO"), co-polyethylene oxide block or random copolymers, and polyvinyl alcohol ("PVA"); poly (vinyl pyrrolidinone) ("PVP"); poly (amino acids); dextran; and proteins such as albumin. The polyethers, such as poly(oxyalkylenes), including polyethylene glycol, may be suitable in some embodiments. When the core is small in molecular nature, any of a variety of hydrophilic functionalities can be used to make the precursor water soluble. For example, functional groups like hydroxyl, amine, sulfonate and carboxylate, which may be water soluble, maybe used to make the precursor water soluble. In addition, while N-hydroxysuccinimide ("NHS") ester of subaric acid is insoluble in water, by adding a sulfonate group to the succinimide ring, the NHS ester of subaric acid may be made water soluble, without affecting its reactivity towards amine groups.

### Biodegradable Linkages

If it is desired that the biocompatible crosslinked polymer be biodegradable or absorbable, one or more precursors having biodegradable linkages present in between the functional groups may be used. The biodegradable linkage may also serve as the water soluble core of one or more of the precursors. In the alternative, or in addition, the functional groups of the precursors may be chosen such that the product of the reaction between them results in a biodegradable linkage. For each approach, biodegradable linkages may be chosen such that the resulting biodegradable biocompatible crosslinked polymer will degrade or be absorbed in a desired period of time. In embodiments, biodegradable linkages may be selected that degrade under physiological conditions into non-toxic products.

The biodegradable linkage may be a component of the small molecule crosslinker, the functional polymer, or both. In embodiments, the reaction of the functional groups of the small molecule crosslinker with the functional groups of the functional polymer may result in the formation of a biodegradable link. The biodegradable linkage may be chemically or enzymatically hydrolyzable or absorbable. Illustrative chemically hydrolyzable biodegradable linkages include polymers, copolymers and oligomers of glycolide, di-lactide, 1-lactide, caprolactone, dioxanone, and trimethylene carbonate. Another chemically hydrolyzable biodegradable linkage that may be utilized is an isolated ester group. As previously stated, an isolated ester group is an ester group that is not adjacent to another ester group. In contrast, polymers and oligomers of, for example, glycolides and lactides, have ester groups that are adjacent to each other. The behavior of an isolated ester group may be distinct from that of non-isolated esters in the context of hydrolysis. Additional illustrative biodegradable linkages that may be utilized include polymers and copolymers of poly(hydroxy acid)s, poly(orthocarbonate)s, poly(anhydride)s, poly(lactone)s, poly(aminoacid)s, poly(carbonate)s, and poly(phosphonate)s.

Illustrative enzymatically biodegradable linkages include peptidic linkages cleavable by proteases, metalloproteinases and collagenases. Cleavage of a polymer by enzymes means that an enzyme has a preference or affinity for a particular chemical group in a polymer as compared to most other chemical groups. In contrast, some enzymes secrete chemicals that exhibit little specificity, for example, enzymes that secrete radicals that degrade the materials that they contact. Further, proteins may generally be considered to be enzymatically cleavable since most proteins have specific sequences that are susceptible to enzymatic degradation. To the contrary, a hydrogel that is resistant to enzymatic degradation does not contain linkages that may be cleaved by enzymes, including metalloproteinases and collagenases.

Biodegradable linkages may be chosen so that a hydrogel is readily degradable. One method of determining that a hydrogel is substantially degraded is to perform an in vitro degradation test, as set forth in Example 13. These tests are generally predictive for degradation in animals, as shown in Examples 13, 15, and 17, as shown in Fig. 46. Fig. 46 shows that in vitro degradation of a trilysine-based hydrogel was about forty days in vitro, and about thirty-eight days in vivo. Thus, for example, a hydrogel may be made using only polymers that have hydrolytically degradable ester groups and no other groups that are enzymatically degradable or degrade by hydrolysis. Other embodiments include hydrogels made using isolated hydrolytically degradable ester groups as the biodegradable linkages. The term "hydrolytically degradable ester group" as used herein means an ester group that degrades spontaneously in aqueous solution; such esters will be hydrolyzed in vitro in water, free of enzymes or bacteria, when kept at about 37° C. In embodiments, the materials may degrade to components that are too small to be observed with the naked eye or small enough to dissolve into aqueous solutions, with the result that the materials seem to disappear over time when exposed to water.

### Visualization Agents

Where convenient, the biocompatible crosslinked polymer, precursor solutions, or both, may contain visualization agents to improve their visibility during surgical procedures. Visualization agents may be especially useful when used in MIS procedures, due among other reasons to their improved visibility on a color monitor.
Visualization agents may be selected from among any of the various non-toxic colored substances suitable for use in medical implantable medical devices, such as FD&C BLUE dyes 3 and 6, eosin, methylene blue, indocyanine green, or colored dyes normally found in synthetic surgical sutures. In embodiments, a color such as green or blue may be desirable, and may have better visibility in the presence of blood or on a pink or white tissue background. For example, red may be hard to visualize when used on a highly vascularized tissue that is red in color. However, red may be suitable when the underlying tissue is white.

The visualization agent may be present with either reactive precursor species, e.g., a crosslinker or functional polymer solution. The colored substance may or may not become chemically bound to the hydrogel. The visualization agent may be used in small quantities, in embodiments less than about 1% weight/volume, in other embodiments less than about 0.01% weight/volume, and in yet other embodiments, less than about 0.001% weight/volume concentration.

Additional visualization agents may be used, such as fluorescent (e.g., green or yellow fluorescent under visible light) compounds (e.g., fluorescein or eosin), x-ray contrast agents (e.g., iodinated compounds) for visibility under x-ray imaging equipment, ultrasonic contrast agents, or MRI contrast agents (e.g., Gadolinium containing compounds).

A visualization agent may be used to allow the user to determine the thickness of the applied hydrogel. The visualization agent may be an agent that provides a color that is visible to the human eye, e.g., a color that is detected visually by the user or detected by a video camera and relayed to a video screen observed by the user.

Wavelengths of light from about 400 to 750 nm are observable to the human as colors (R.K. Hobbie, Intermediate Physics for Medicine and Biology, 2nd Ed., pages 371-373). Blue color is perceived when the eye receives light that is predominantly from about 450 to 500 nm in wavelength and green is perceived at about 500 to 570 nm (Id.). The color of an object is therefore determined by the predominant wavelength of light that it reflects or emits. Further, since the eye detects red or green or blue, a combination of these colors may be used to simulate any other color merely by causing the eye to receive the proportion of red, green, and blue that is perceived as the desired color by the human eye. Blue and green visualization agents may be useful since they are most readily visible when observing in situ crosslinking due to the approximately red color of the background color of tissue and blood. The color blue, as used herein, means the color that is perceived by a normal human eye stimulated by a wavelength of about 450 to 500 nm and the color green, as used herein, means the color that is perceived by a normal human eye stimulated by a wavelength of about 500 to 570 nm.

The use of a visualization agent may be used when a hydrogel is introduced into a polyp or the submucosa of a polyp. The hydrogel forms as the precursors crosslink after contacting the substrate surface, i.e., the polyp or submucosa of a polyp, which allows the hydrogel precursors to mix and conform to the shape of the polyp.

In embodiments, a hydrogel of the present disclosure for use on a patient's tissue may include water, a biocompatible visualization agent, and crosslinked hydrophilic polymers that form a hydrogel after contact with the tissue. The hydrogel forms on or in the tissue, such as a polyp. The visualization agent reflects or emits light at a wavelength detectable to a human eye so that a user applying the hydrogel can observe the gel and also visualize the gel in a polyp as portions of the polyp are removed.

Some suitable biocompatible visualization agents include FD&C BLUE #1, FD&C BLUE #2, and methylene blue. These agents may be present in the final electrophilic-nucleophilic reactive precursor species mix at a concentration of more than about 0.05 mg/ml, in a concentration of from about 0.1 to about 12 mg/ml, in embodiments from about 0.1 to about 4 mg/ml, although greater concentrations may potentially be used, up to the limit of solubility of the visualization agent. The visualization agent may also be a fluorescent molecule.

### Crosslinking Reactions

The crosslinking reactions may occur in aqueous solution under physiological conditions. In embodiments, the crosslinking reactions may occur "in situ", meaning they occur at local sites such as on or in organs or tissues in a living animal or human body, including polyps and the submucosa adjacent a polyp. In embodiments, the crosslinking reactions do not release heat of polymerization. The crosslinking reaction leading to gelation may occur within about 10 minutes, in embodiments within about 2 minutes, in other embodiments within about one minute, and in yet other embodiments within about 30 seconds. In some embodiments, the formation of a biocompatible crosslinked polymer of the present disclosure may occur within about 45 seconds, as measured by a gel time measurement.

Certain functional groups, such as alcohols or carboxylic acids, do not normally react with other functional groups, such as amines, under physiological conditions (e.g., pH 7.2-11, 37°C). However, such functional groups can be made more reactive by using an activating group such as N-hydroxysuccinimide. Several methods for activating such functional groups are within the purview of those skilled in the art. Suitable activating groups include carbonyldiimidazole, sulfonyl chloride, aryl halides, sulfosuccinimidyl esters, N-hydroxysuccinimidyl ester, succinimidyl ester, epoxide, aldehyde, maleimides, imidoesters and the like. In embodiments, the N-hydroxysuccinimide esters or N-hydroxysulfosuccinimide groups may be utilized for crosslinking of proteins or amine functionalized polymers such as amino terminated polyethylene glycol ("APEG").

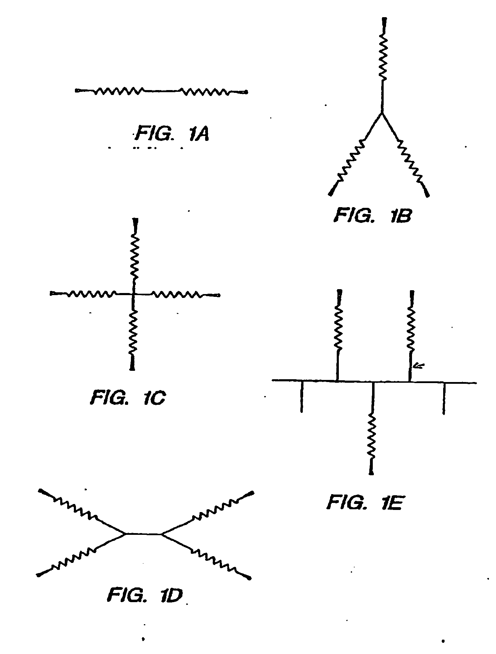

FIGS. 1 to 5 illustrate various embodiments of suitable crosslinkers and functional polymers. FIG. 1 illustrates possible configurations of degradable electrophilic crosslinkers or functional polymers. The biodegradable regions are represented by (^^^^^^^); the functional groups are represented by ( ◄ ) and the inert water soluble cores are represented by (-). For crosslinkers, the central core is a water soluble small molecule, and for functional polymers the central core is a water soluble polymer of natural or synthetic origin.

When Structure A in FIG. 1 is a functional polymer, it is a linear water soluble and biodegradable functional polymer, end-capped with two functional groups (e.g., N-hydroxysuccinimide ester or NHS, epoxide or similar reactive groups). The water soluble core may be a polyalkylene oxide, for example a polyethylene glycol block copolymer, and it is extended with at least one biodegradable linkage between it and each terminal functional group. The biodegradable linkage may be a single linkage or copolymers or homopolymers of absorbable polymers such as polyhydroxy acids or polylactones.

When Structure B in FIG. 1 is a functional polymer, it is a branched or star shaped biodegradable functional polymer which has an inert polymer at the center. The inert and water soluble core may be terminated with oligomeric biodegradable extensions, which in turn may be terminated with reactive functional groups.

When Structures C and D in FIG. 1 are functional polymers, they may be multifunctional 4 arm biodegradable functional polymers. This polymer again has a water-soluble core at the center, which is a 4 arm, tetrafunctional polyethylene glycol (Structure C) or block copolymer of PEO-PPO-PEO such as TETRONIC 908 (Structure D), which is extended with small oligomeric extensions of at least one biodegradable polymer to maintain water solubility and terminated with reactive functional end-groups such as CDI or NHS.

When Structure E in FIG. 1 is a functional polymer, it is a multifunctional star or graft type biodegradable polymer. This polymer has a water-soluble polymer like polyethylene oxide, polyvinyl alcohol or poly(vinyl pyrrolidinone) at the core, which is completely or partially extended with biodegradable polymer. The biodegradable polymer is terminated with reactive end groups.

Structures A-E in FIG. 1 need not have polymeric cores and may be small molecule crosslinkers. In that case, the core may include a small molecule like ethoxylated glycerol, inositol, trimethylolpropane etc. to form the resultant crosslinker. In addition, Structures A-E in FIG. 1 need not have polymeric biodegradable extensions, and the biodegradable extensions may include small molecules like succinate or glutarate or combinations of 2 or more esters, such as glycolate/2-hydroxybutyrate or glycolate/4-hydroxyproline, etc. A dimer or trimer of 4-hydroxyproline may be used, not only to add degradability, but also to add nucleophilic functional group reactive sites via the pendant primary amines which may be part of the hydroxyproline moiety.

Other variations of the core, the biodegradable linkage, and the terminal electrophilic group in Structures A-E in FIG. 1 may be constructed, so long as the resulting functional polymer has the properties of low tissue toxicity, water solubility, and reactivity with nucleophilic functional groups.

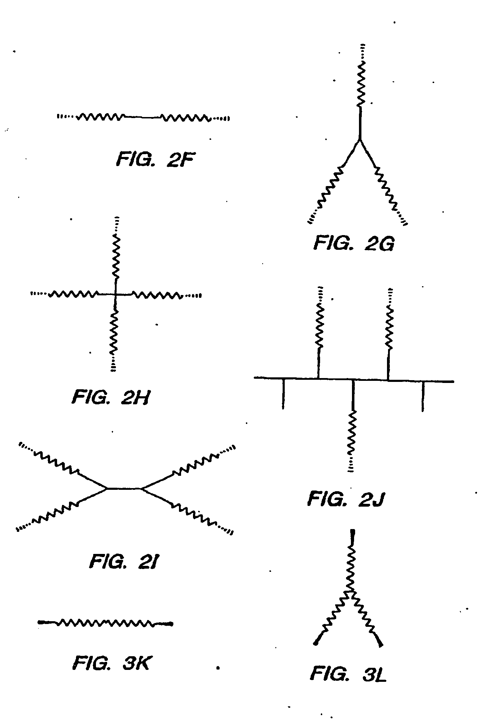

FIG. 2 illustrates various embodiments of nucleophilic biodegradable water soluble crosslinkers, and functional polymers suitable for use with electrophilic functional polymers and crosslinkers described herein. The biodegradable regions are represented by (^^^^^^^); the functional groups are represented by ( | ); and the inert water soluble cores are represented by (-). For crosslinkers, the central core is a water soluble small molecule and for functional polymers the central core is a water soluble polymer of natural or synthetic origin. When Structure F in FIG. 2 is a functional polymer, it is a linear water soluble biodegradable polymer terminated with reactive functional groups like primary amine. The linear water-soluble core may be a polyalkylene oxide, such as a polyethylene glycol block copolymer, which is extended with the biodegradable region which may be a copolymer or homopolymers of polyhydroxy acids or polylactones. This biodegradable polymer may be terminated with primary amines. When Structure G in FIG. 2 is a functional polymer, it is a branched or star shaped biodegradable polymer which has an inert polymer at the center. The inert polymer may be extended with single or oligomeric biodegradable extensions which may be terminated with reactive functional groups. When Structures H and I in FIG. 2 are functional polymers, they may be multifunctional 4 arm biodegradable polymers. These polymers again have water-soluble cores at their center which may be either a 4 arm, tetrafunctional polyethylene glycol (Structure H) or a block copolymer of PEO-PPO-PEO such as TETRONIC 908 (Structure I), extended with small oligomeric extensions of biodegradable polymers to maintain water solubility, and terminated with functional groups such as amines and thiols.

When Structure J in FIG. 2 is a functional polymer, it may be a multifunctional star or graft type biodegradable polymer. This polymer has a water soluble polymer like polyethylene oxide, polyvinyl alcohol or poly(vinyl pyrrolidinone) at the core which may be completely or partially extended with biodegradable polymer. The biodegradable polymer may be terminated with reactive end groups. Structures F-J in FIG. 2 need not have polymeric cores and may be small molecule crosslinkers. In that case, the core may include a small molecule like ethoxylated glycerol, inositol, trimethylolpropane, and the like, to form the resulting crosslinker. Other variations of the core, the biodegradable linkage, and the terminal nucleophilic functional group in Structures F-J in FIG. 2 may be constructed, so long as the resulting functional polymer has the properties of low tissue toxicity, water solubility, and reactivity with electrophilic functional groups.

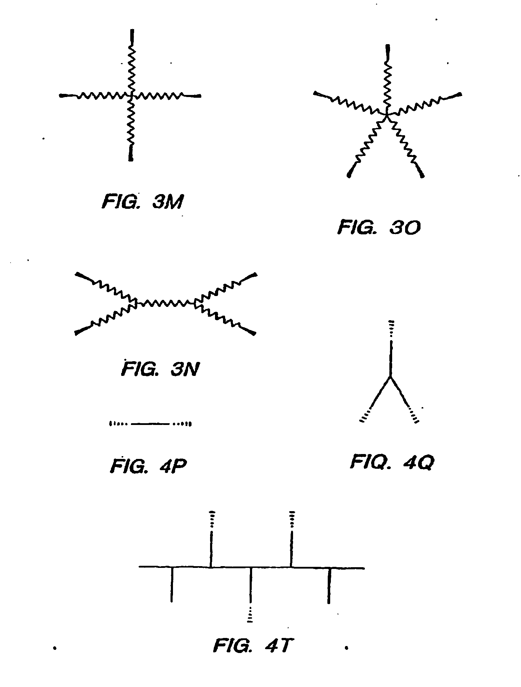

FIG. 3 illustrates configurations of water-soluble electrophilic crosslinkers or functional polymers where the core is biodegradable. The biodegradable regions are represented by (^^^^^^^) and the functional groups are represented by ( ◄ ). The biodegradable core may be terminated with a reactive functional group that is also water solubilizing, such a N-hydroxysulfosuccinimide ester ("SNHS") or N-hydroxyethoxylated succinimide ester ("ENHS"). Structure K in FIG. 3 depicts a difunctional biodegradable polymer or oligomer terminated with SNHS or ENHS. The oligomers and polymers may be made of a poly(hydroxy acid) such as poly(lactic acid), which is insoluble in water. However, the terminal carboxylic acid group of these oligomers or polymers can be activated with N-hydroxysulfosuccinimide ester ("SNHS") or N-hydroxyethoxylated succinimide ester ("ENHS") groups. An ionic group, like a metal salt (such as a sodium salt) of sulfonic acid, or a nonionic group, like a polyethylene oxide on the succinimide ring, provides water-solubility while the NHS ester provides chemical reactivity towards amines. The sulfonate groups (sodium salts) or ethoxylated groups on the succinimide ring solubilize the oligomer or polymer without appreciably inhibiting reactivity towards amine groups. Structures L-O in FIG. 3 represent multi-branched or graft type structures with terminal SNHS or ENHS groups. The cores may include various non-toxic polyhydroxy compounds like sugars (xylitol, erythritol), glycerol, and/or trimethylolpropane, which have been reacted with anhydrides such as succinic or glutaric anhydrides. The resultant acid groups were then activated with SNHS or ENHS groups to form water soluble crosslinkers or functional polymers.

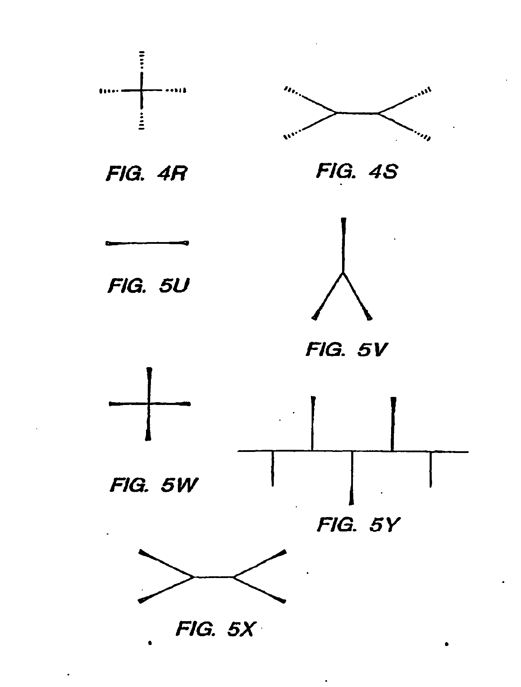

FIG. 4 illustrates various nucleophilic functional polymers or crosslinkers that are not biodegradable. The nucleophilic functional groups may be represented by ( | ) and the inert water-soluble cores may be represented by (-). For crosslinkers, the central core may be a water-soluble small molecule and for functional polymers the central core may be a water soluble polymer of natural or synthetic origin. When Structure P in FIG. 4 is a functional polymer, it may be a water-soluble linear polymer such as polyethylene glycol terminated with reactive end group such as primary amines and thiols. Such polymers include those commercially available from Sigma (Milwaukee, WI) and Shearwater Polymers (Huntsville, AL). Some other suitable difunctional polymers include PPO-PEO-PPO block copolymers such as PLURONIC F68 terminated with amine groups. PLURONIC or TETRONIC polymers may be available with terminal hydroxyl groups. The hydroxyl groups may be converted into amine groups by methods within the purview of those skilled in the art. Structures Q-T in FIG. 4 include functional polymers that may be multifunctional graft or branch type water soluble copolymers with terminal amine groups. Structures P-T in FIG. 4 need not have polymeric cores and may be small molecule crosslinkers. In that case, the core may include a small molecule like ethoxylated glycerol, inositol, trimethylolpropane, dilysine etc. to form the resultant crosslinker. Other variations of the core and the terminal nucleophilic functional group in Structure P-T in FIG. 4 may be employed, so long as the properties of low tissue toxicity, water solubility, and reactivity with electrophilic functional groups are maintained.

FIG. 5 illustrates various electrophilic functional polymers or crosslinkers that are not biodegradable. The electrophilic functional groups are represented by ( | ) and the inert water soluble cores are represented by (-). For crosslinkers, the central core may be a water soluble small molecule and for functional polymers the central core may be a water soluble polymer of natural or synthetic origin. When Structure U is a functional polymer, it may be a water-soluble polymer such as polyethylene glycol terminated reactive end group such as NHS or epoxide. Such polymers include those commercially available from Sigma and Shearwater polymers. Some other suitable polymers include PPO-PEO-PPO block copolymers such as PLURONIC F68 terminated with NHS or SNHS group. As noted above, PLURONIC or TETRONIC polymers may be available with terminal hydroxyl groups. The hydroxyl groups may be converted into acid group by reacting with succinic anhydride. The terminated acid groups may be reacted with N-hydroxysuccinimide in presence of DCC to generate NHS activated PLURONIC polymer. When Structures V-Y are functional polymers they may be multifunctional graft or branch type PEO or PEO block copolymers (TETRONICS) activated with terminal reactive groups such as NHS. Structures U-Y in FIG. 5 need not have polymeric cores and may be small molecule crosslinkers. In that case, the core may comprise a small molecule like ethoxylated glycerol, tetraglycerol, hexaglycerol, inositol, trimethylolpropane, dilysine etc. to form the resultant crosslinker. Other variations of the core and the terminal nucleophilic functional group in Structures U-Y in FIG. 5 may be employed, so long as the properties of low tissue toxicity, water solubility, and reactivity with electrophilic functional groups may be maintained.

### Preparation of Structures A-Y in FIGS. 1-5

The polymeric crosslinkers and functional polymers illustrated as Structures A-Y in FIGS. 1 to 5 may be prepared using variety of synthetic methods. Exemplary compositions include those described in Table 1.

**Table 1.**

| Suitable Crosslinkers and Functional Polymers | | |
|---|---|---|
| Structure | Brief Description | Example |
| A | Water soluble, linear difunctional crosslinker or functional polymer with water soluble core, extended with biodegradable regions such as oligomers of hydroxyacids or peptide sequences which are cleavable by enzymes and terminated with protein reactive functional groups | Polyethylene glycol or ethoxylated propylene glycol chain extended with oligolactate and terminated with N-hydroxysuccinimide esters |
| B | Water soluble, trifunctional crosslinker or functional polymer with water soluble core, extended with biodegradable regions such as oligomers of hydroxyacids or peptide sequences and terminated with protein reactive functional groups | Ethoxylated glycerol chain extended with oligolactate and terminated with N-hydroxysuccinimide esters |
| C | Water soluble, tetrafunctional crosslinker or functional polymer with water soluble core, extended with biodegradable regions such as oligomers of hydroxyacids or peptide sequences and terminated with protein reactive functional groups | 4 arm polyethylene glycol, erythritol or pentaerythritol or pentaerythritol chain extended with oligolactate and terminated with N-hydroxysuccinimide esters |
| D | Water soluble, tetrafunctional crosslinker or functional polymer with water soluble core, extended with biodegradable regions such as oligomers of hydroxyacids or peptide sequences and terminated with protein reactive functional groups | Ethoxylated ethylene diamine or polyethylene oxide-polypropylene oxide-polyethylene oxide block copolymer like TETRONIC 908 chain extended with oligotrimethylene carbonate and terminated with N-hydroxysuccinimide ester |
| E | Water soluble, branched crosslinker or functional polymer with water soluble core, extended with biodegradable regions such as oligomers of hydroxyacids or peptide sequences and terminated with protein reactive functional groups | Low molecular weight polyvinyl alcohol with 1 % to 20% hydroxyl groups extended with oligolactate and terminated with N-hydroxysuccinimide ester |
| F | Water soluble, liner difunctional crosslinker or functional polymer with water soluble core, extended with biodegradable regions such as oligomers of hydroxyacids or peptide sequences and terminated with amines, carboxylic acid or thiols | Polyethylene oxide-polypropylene oxide-polyethylene oxide block copolymer surfactant like PLURONIC F68 chain extended with oligolactate and terminated with amino acids such as lysine or peptide sequences that may contain two amine groups |
| G | Water soluble, trifunctional crosslinker or functional polymer with water soluble core, extended with biodegradable regions such as oligomers of hydroxyacids or peptide sequences and terminated with amines, carboxylic acid or thiols | Ethoxylated glycerol chain extended with oligolactate and terminated with aminoacid such as lysine |
| H | Water soluble, tetrafunctional crosslinker or functional polymer with water soluble core, extended with biodegradable regions such as oligomers of hydroxyacids or peptide sequences and terminated with amines, carboxylic acid or thiols | 4 arm polyethylene glycol or tetra erythritol chain extended with oligolactate and terminated with aminoacid such as lysine |
| I | Water soluble, tetrafunctional crosslinker or functional polymer with water soluble core, extended with biodegradable regions such as oligomers of hydroxyacids or peptide sequences and terminated with amines, carboxylic acid or thiols | Ethoxylated ethylene diamine or polyethylene oxide-polypropylene oxide-polyethylene oxide block copolymer like TETRONIC 908 chain extended with oligotrimethylene carbonate and terminated with aminoacid such as lysine |
| J | Water soluble, multifunctional or graft type crosslinker or functional polymer with water soluble core, extended with biodegradable regions such as oligomers of hydroxyacids or peptide sequences and terminated with amines, carboxylic acid or thiols | Low molecular weight polyvinyl alcohol with 1-20% hydroxyl groups extended with oligolactate and terminated with aminoacid such as lysine |
| K | Water soluble, linear difunctional crosslinker or functional polymer such as oligomers of hydroxyacids or peptide sequences which are terminated with protein reactive functional groups | Difunctional oligolactic acid with terminal carboxyl groups which are activated with n-hydroxysulfosuccinimide ester or ethoxylated n-hydroxysuccinimide ester. |
| L | Water soluble branched trifunctional crosslinker or functional polymer such as oligomers of hydroxyacids or peptide sequences which are terminated with protein reactive functional groups | Trifunctional oligocaprolactone with terminal carboxyl groups which are activated with n-hydroxysulfosuccinimide ester or ethoxylated n-hydroxysuccinimide ester. |
| M | Water soluble, branched tetrafunctional crosslinker or functional polymer such as oligomers of hydroxyacids or peptide sequences which are terminated with protein reactive functional groups | Tetrafunctional oligocaprolactone with terminal carboxyl groups which are activated with n-hydroxysulfosuccinimide ester or ethoxylated n-hydroxysuccinimide ester. |
| N | Water soluble, branched tetrafunctional crosslinker or functional polymer such as oligomers of hydroxyacids or peptide sequences which are terminated with protein reactive functional groups | Tetrafunctional oligocaprolactone with terminal carboxyl groups which are activated with n-hydroxysulfosuccinimide ester or ethoxylated n-hydroxysuccinimide ester. |
| O | Water soluble, branched multifunctional crosslinker or functional polymer such as oligomers f hydroxyacids or peptide sequences which are terminated with protein reactive functional groups | Multifunctional oligolactic acid with terminal carboxyl groups which are activated with n-hydroxysulfosuccinimide ester or ethoxylated n-hydroxysuccinimide ester. |
| P | Water soluble, linear difunctional crosslinker or functional polymer terminated with amines, carboxylic acid or thiols functional groups | Polyethylene glycol with terminal amines groups |
| Q | Water soluble, branched trifunctional crosslinker or functional polymer terminated with amines, carboxylic acid or thiols as functional group | Ethoxylated glycerol with terminal amines groups |
| R | Water soluble, branched tetrafunctional crosslinker of functional polymer terminated with amines, carboxylic acid or thiols functional groups | 4 arm polyethylene glycol modified to produce terminal amine groups |
| S | Water soluble, branched tetrafunctional crosslinker or functional polymer terminated with amines, carboxylic acid or thiols functional groups | Ethoxylated ethylene diamine or polyethylene oxide-polypropylene oxide-polyethylene oxide block copolymer like TETRONIC 908 modified to generate terminal amine groups |
| T | Water soluble, branched or graft crosslinker or functional polymer with terminal amines, carboxylic acid or thiols functional groups | Polylysine, albumin, polyallyl amine |
| U | Water soluble, linear difunctional crosslinker or functional polymer terminated with protein reactive functional groups | Polylysine, albumin, polyallyl amine |
| V | Water soluble branched trifunctional crosslinker or functional polymer terminated with protein reactive functional groups | Ethoxylated glycerol terminated with n-hydroxysuccinimide |
| W | Water soluble branched tetrafunctional crosslinker or functional polymer terminated with protein reactive functional groups | 4 arm polyethylene glycol terminated with n-hydroxysuccinimide esters |
| X | Water soluble branched tetrafunctional crosslinker or functional polymer terminated with protein reactive functional groups | Ethoxylated ethylene diamine or polyethylene oxide-polypropylene oxide-polyethylene oxide block copolymer like TETRONIC 908 with n-hydroxysuccinimide ester as end group |
| Y | Water soluble, branched or graft polymer crosslinker or functional polymer with protein reactive functional groups | Poly (vinyl pyrrolidinone)-co-poly (n-hydroxysuccinimide acrylate) copolymer (9: 1), molecular weight < 40000 Da |

First, the biodegradable links of Structures A-J in FIGS. 1 and 2 may be composed of specific di or multifunctional synthetic amino acid sequences which may be recognized and cleaved by enzymes such as collagenase, and may be synthesized using methods within the purview of those skilled in the peptide synthesis art. For example, Structures A-E in FIG. 1 may be obtained by first using carboxyl, amine or hydroxy terminated polyethylene glycol as a starting material for building a suitable peptide sequence. The terminal end of the peptide sequence may be converted into a carboxylic acid by reacting succinic anhydride with an appropriate amino acid. The acid group generated may be converted to an NHS ester by reaction with N-hydroxysuccinimide.

The functional polymers described in FIG. 2 may be prepared using a variety of synthetic methods. In embodiments, the polymer shown as Structure F may be obtained by ring opening polymerization of cyclic lactones or carbonates initiated by a dihydroxy compound such as PLURONIC F 68 in the presence of a suitable catalyst such as stannous 2-ethylhexanoate. The molar equivalent ratio of caprolactone to PLURONIC is kept below 10 to obtain a low molecular weight chain extension product so as to maintain water solubility. The terminal hydroxyl groups of the resultant copolymer may be converted into amine or thiol by methods within the purview of those skilled in the art.

In embodiments, the hydroxyl groups of a PLURONIC-caprolactone copolymer may be activated using tresyl chloride. The activated groups may then be reacted with lysine to produce lysine terminated PLURONIC-caprolactone copolymer. Alternatively, an amine-blocked lysine derivative is reacted with the hydroxyl groups of a PLURONIC-caprolactone copolymer and then the amine groups may be regenerated using a suitable deblocking reaction.
Structures G, H, I and J in FIG. 2 may represent multifunctional branched or graft type copolymers having a water soluble core extended with oligohydroxy acid polymer and terminated with amine or thiol groups.

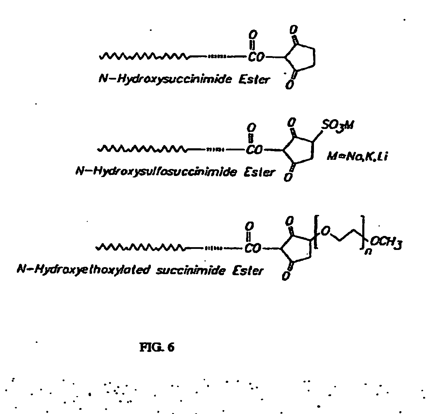

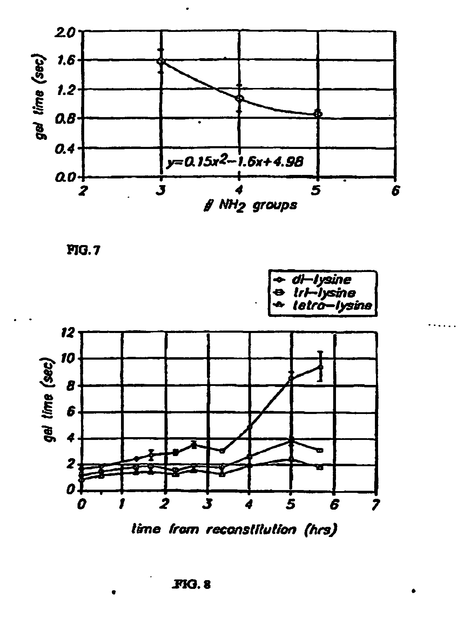

For example, in embodiments, the functional polymer illustrated as Structure G in FIG. 2 may be obtained by ring opening polymerization of cyclic lactones or carbonates initiated by a tetrahydroxy compound, such as a 4 arm, tetrahydroxy polyethylene glycol (molecular weight 10,000 Da), in the presence of a suitable catalyst such as stannous octoate. The molar equivalent ratio of cyclic lactone or carbonate to PEG is kept below about 10 to obtain a low molecular weight extension, and to maintain water solubility (polymers of cyclic lactones generally are not as water soluble as PEG). Alternatively, a hydroxyacid as a biodegradable link may be attached to the PEG chain using blocking/deblocking chemistry within the purview of those skilled in the peptide synthesis art. The terminal hydroxy groups of the resultant copolymer may be activated using a variety of reactive groups within the purview of those skilled in the art. The CDI activation chemistry and sulfonyl chloride activation chemistry is shown in FIGS. 6 and 7, respectively, of U.S. Patent No. 7,009,056, the entire disclosure of which is incorporated by reference herein.

In embodiments, N-hydroxysuccinimide esters may be utilized as the reactive groups, which may be synthesized by any of several methods. For example, hydroxyl groups may be converted to carboxylic groups by reacting them with anhydrides such as succinic anhydride in the presence of tertiary amines such as pyridine or triethylamine or dimethylaminopyridine ("DMAP"). Other anhydrides such as glutaric anhydride, phthalic anhydride, maleic anhydride, and the like, may also be used. The resultant terminal carboxyl groups may be reacted with N-hydroxysuccinimide in the presence of dicyclohexylcarbodiimide ("DCC") to produce N-hydroxysuccinimide ester (referred to herein as NHS activation). Suitable N-hydroxysuccinimide esters are shown in FIG. 6.

In embodiments, the polymer shown as structure H is obtained by ring opening polymerization of glycolide or trimethylene carbonate initiated by a tetrahydroxy compound such as tetrafunctional polyethylene glycol (molecular weight about 2000 Da) in the presence of a catalyst such as stannous 2-ethylhexoate. The molar equivalent ratio of glycolide to PEG is kept from about 2 to about 10 to obtain a low molecular weight extension. The terminal hydroxy groups of the resultant copolymer may be converted into amine groups by reaction with lysine as previously mentioned. Similar embodiments can be obtained using analogous synthetic chain extension strategies to obtain structures F, G, I and J by starting with the appropriate corresponding polyol.

Structures K, L, M, N and O in FIG. 3 may be made using a variety of synthetic methods. In embodiments, the polymer shown as Structure L in FIG. 3 may be obtained by ring opening polymerization of cyclic lactones by a trihydroxy compound such as glycerol in the presence of a catalyst such as stannous 2-ethylhexanoate. The molar equivalent ratio of cyclic lactone to glycerol is kept below 2, so that only low molecular weight oligomers are obtained. The low molecular weight oligomer ester is insoluble in water. The terminal hydroxy groups of the resultant copolymer may be activated using N-hydroxysulfosuccinimide groups. This is achieved by converting hydroxy groups to carboxylic groups by reacting with anhydrides such as succinic anhydride in the presence of tertiary amines. The resulting terminal carboxyl groups may be reacted with N-hydroxysulfosuccinimide or N-hydroxyethoxylated succinimide in the presence of dicyclohexylcarbodiimide ("DCC") to produce a sulfonated or ethoxylated NHS ester. The sulfonate or PEO chain on the succinimide ring provides water solubility to the oligoester.

The foregoing method generally is applied to solubilize only low molecular weight multi-branched oligoesters, with molecular weights below 1000. In another variation of this method, various non-toxic polyhydroxy compounds, for example sugars, such as erythritol and/or xylitol, may be reacted with succinic anhydride in the presence of a tertiary amine. The terminal carboxyl group of succinated erythritol may then be esterified with N-hydroxysulfosuccinimide (FIG. 6). Similar embodiments may be obtained using analogous synthetic strategies to obtain structures K, and M-O by starting with the appropriate starting materials.

Structures P-R may be synthesized by reacting the appropriate starting material, such as a linear (structure P) or 2- or 3-arm branched PEG (structures Q, R) with hydroxy end groups, with lysine as mentioned previously, such that the arms of the PEG oligomers may be capped with amine end groups. Structure S may be synthesized, using a multistep reaction, from PEG, glycerol and a diisocyanate. In the first step, a PEG diol may be reacted with excess diisocyanate, such as 4, 4'diphenyl methane diisocyanate ("MDI"), methylene-bis (4-cyclohexylisocyanate) ("HMDI") or hexamethylenediisocyanate ("HDI"). After purification, the resultant PEG diisocyanate is added dropwise to excess glycerol, or trimethylol propane or other triol, and reacted to completion. The purified product, now having diol end groups, is again reacted with excess diisocyanate and purified, yielding a PEG-tetra-isocyanate. This tetrafunctional PEG may be subsequently reacted with excess PEG diols, yielding a 4 arm PEG synthesized from a PEG diol oligomer. In the final step, lysine end groups may be incorporated, as discussed previously.

Structure T may be synthesized as follows: a random copolymer of PEG-monoacrylate and some other acrylate or combination of acrylates may be synthesized, such that the final polyacrylate is water soluble. Other acrylates include, but are not limited to, 2-hydroxyethylacrylate, acrylic acid, and acrylamide. Conditions may be varied to control the molecular weight as desired. In the final step, the acrylate may be reacted with lysine as discussed previously, using an appropriate quantity to achieve the desired degree of amination.

One method of synthesizing Structures U-Y is to use dicyclohexylcarbodiimide coupling to a carboxylate end group. For Structures U-W, one can react the appropriate PEG-diol, -triol or -tetra-hydroxy starting material with excess succinic anhydride or glutaric anhydride such that all end groups are effectively carboxylated. Structures X and Y may be made in a manner similar to that used for Structures S and T, except that in the last step, instead of end capping with lysine, end capping with succinic anhydride or glutaric anhydride is performed.

### Preparation of Biocompatible Polymers

Several biocompatible crosslinked hydrogels may be produced using the crosslinkers and functional polymers described in FIGS. 1 to 5. Exemplary combinations of such polymers for producing such biocompatible crosslinked polymers are described in Table 2. In Table 2, the crosslinker functional groups include N-hydroxy succinimide esters and the functional polymer functional groups include primary amines.

**Table 2.**

| Biocompatible Polymers Synthesized from Crosslinkers and Functional Polymers of Table 1 | | | |
|---|---|---|---|
| Crosslinker Structure | Functional Polymer Structure | Concentration | Medium |
| B or C | H and R | Molar Equivalent; > 20% W/V | Borate or triethanol amine buffer, pH 7-10 |
| A, B or C | H, P, Q, R and S | Molar Equivalent; > 20% W/V | Borate or triethanol amine buffer, pH 7-10 |
| Y | T, H, P and Q | Molar Equivalent; > 10% W/V | Borate or triethanol amine buffer, pH 7-10 |
| W, V | H and J | Molar Equivalent; > 20% W/V | Bicarbonate buffer, pH 7-10 |
| X | I, J and H | Molar Equivalent; > 20% W/V | Borate or triethanol amine buffer, pH 7-10 |

The reaction conditions for crosslinking may depend on the nature of the functional groups. Exemplary reactions may be conducted in buffered aqueous solutions at a pH of from about 5 to about 12. Suitable buffers include sodium borate buffer (pH about 10) and triethanol amine buffer (pH about 7). Elevated pH increases the speed of electrophilic-nucleophilic reactions. In some embodiments, organic solvents such as ethanol or isopropanol may be added to improve the reaction speed or to adjust the viscosity of a given formulation.

Many of the synthetic crosslinked gels described herein degrade due to hydrolysis of the biodegradable region, especially by hydrolysis of the isolated ester in the polymer. The degradation of gels containing synthetic peptide sequences will depend on the specific enzyme capable of degrading the peptide and its concentration. In some cases, a specific enzyme may be added during the crosslinking reaction to accelerate the degradation process.

When the crosslinker and functional polymers are synthetic (for example, when they are based on polyalkylene oxide), it may be desirable to use molar equivalent quantities of the reactants. In some cases, molar excess crosslinker may be added to compensate for side reactions, such as reactions due to hydrolysis of the functional group.

When choosing the crosslinker and crosslinkable polymer, at least one of the precursors should have more than 2 functional groups per molecule and at least one degradable region, if it is desired that the resultant biocompatible crosslinked polymer be biodegradable. For example, the difunctional crosslinker shown as Structure A in FIG. 1 cannot form a crosslinked network with the difunctional polymers shown as Structure F in FIG. 2 or Structure P in Fig. 4. Generally, it may be desirable that each biocompatible crosslinked polymer precursor have more than 2, in embodiments 4 or more, functional groups.

In embodiments, the crosslinker may have n crosslinker functional groups, wherein n is two or more, and the synthetic functional polymer may have m functional polymer functional groups, wherein m is two or more, and the sum of n and m is five or more. Thus, where one of the precursors possesses two functional groups, the other precursor should posses at least 3 functional groups. In other embodiments, where the functional polymer functional groups are nucleophilic, the crosslinker functional groups may be electrophilic, and where the functional polymer functional groups are electrophilic, the crosslinker functional groups may be nucleophilic. Thus, in embodiments, the crosslinker functional groups may be different than the functional polymer functional groups.

Suitable electrophilic functional groups include NHS, SNHS and ENHS (FIG. 6). Suitable nucleophilic functional groups include primary amines. The advantage of the NHS-amine reaction is that the reaction kinetics lead to quick gelation, usually within about 10 minutes, in embodiments within about 1 minute, and in other embodiments within about 10 seconds. This fast gelation may be desirable for in situ reactions on or in live tissue.

The NHS-amine crosslinking reaction leads to formation of N-hydroxysuccinimide as a side product. The sulfonated or ethoxylated forms of N-hydroxysuccinimide may be desirable due to their increased solubility in water and hence their rapid clearance from the body. The sulfonic acid salt on the succinimide ring does not alter the reactivity of the NHS group with the primary amines.

The NHS-amine crosslinking reaction may be carried out in aqueous solutions and in the presence of buffers. Suitable buffers include phosphate buffer (pH 5-7.5). triethanolamine buffer (pH 7.5-9), borate buffer (pH 9-12), and sodium bicarbonate buffer (pH 9-10). Aqueous solutions of NHS based crosslinkers and functional polymers may be made just before the crosslinking reaction due to reaction of NHS groups with water. Longer "pot life" may be obtained by keeping these solutions at lower pH (pH 4-5). The crosslinking density of the resultant biocompatible crosslinked polymer may be controlled by the overall molecular weight of the crosslinker and functional polymer and the number of functional groups available per molecule. A lower molecular weight between crosslinks such as 600 will give much higher crosslinking density as compared to a higher molecular weight such as 10,000. Higher molecular weight functional polymers may be desirable, in embodiments having a molecular weight greater than about 3000, so as to obtain elastic gels. The crosslinking density may also be controlled by the overall percent solids of the crosslinker and functional polymer solutions. Increasing the percent solids increases the probability that an electrophilic functional group will combine with a nucleophilic functional group prior to inactivation by hydrolysis. Yet another method to control crosslink density is by adjusting the stoichiometry of nucleophilic functional groups to electrophilic functional groups. A one to one ratio leads to the highest crosslink density.

In embodiments, the concentration of solids in the biocompatible crosslinked polymer of the present disclosure, which may be a hydrogel, may be from about 8 percent by weight to about 20 percent by weight.

Natural polymers, for example proteins or glycosaminoglycans, e.g., collagen, fibrinogen, albumin, and fibrin, may be crosslinked using reactive precursor species with electrophilic functional groups. Natural polymers may be proteolytically degraded by proteases present in the body. Synthetic polymers and reactive precursor species may be desirable, however, and may have electrophilic functional groups that include carbodiimidazole, sulfonyl chloride, chlorocarbonates, n-hydroxysuccinimidyl esters, succinimidyl esters, or sulfosuccinimidyl esters. The term synthetic means a molecule that is not found in nature, e.g., polyethylene glycol. The nucleophilic functional groups may be, for example, amine, hydroxyl, carboxyl, and thiol. The polymers may also have a polyalkylene glycol portion, including polyethylene glycol. The polymers may also have a hydrolytically biodegradable portion or linkage, for example an ester, carbonate, or an amide linkage. Several such linkages are within the purview of those skilled in the art and originate from alpha-hydroxy acids, their cyclic dimers, or other chemical species used to synthesize biodegradable articles, such as, glycolide, dl-lactide, I-lactide, caprolactone, dioxanone, trimethylene carbonate or a copolymer thereof. Another embodiment has reactive precursor species with two to ten nucleophilic functional groups each, and reactive precursor species with two to ten electrophilic functional groups each. The hydrophilic species may be synthetic molecules.

In embodiments, biocompatible crosslinked polymers may be formed from the reaction of precursors having electrophilic functional groups and nucleophilic functional groups. The precursors may be water soluble, non-toxic and biologically acceptable. In embodiments, at least one of the precursors is a small molecule having a molecular weight of about 2000 Da or less, or about 1000 Da or less, and may be referred to as a "crosslinker". The crosslinker may have a solubility of at least 1 g/100 mL in an aqueous solution. A crosslinked molecule may be crosslinked via an ionic or covalent bond, a physical force, or other attraction. At least one of the other precursors may be a macromolecule, and may be referred to as a "functional polymer". The functional polymer, when reacted in combination with a crosslinker, may be from at least about five to about fifty times greater in molecular weight than the small molecule crosslinker, and less than about 60,000 Da in some embodiments. In other embodiments, the functional polymer may be from about seven to about thirty times greater in molecular weight than the crosslinker, and in yet other embodiments the functional polymer may be from about ten to about twenty times greater in molecular weight than the crosslinker. In some embodiments the molecular weight of the functional polymer may be at least 7 times more than the crosslinker.

In some embodiments the composition of the present disclosure may include at least one biocompatible crosslinker region including a crosslinked synthetic crosslinker molecule having a pre-crosslinked molecular weight of less than about 2000, in combination with at lest one biocompatible functional polymer region possessing a crosslinked synthetic polymer molecule having a pre-crosslinked molecular weight of more than about 7 times the molecular weight of the pre-crosslinked crosslinker molecule. In such an embodiment, the crosslinker and the functional polymer may react in tissue, for example the submucosa of a polyp, to form a biocompatible crosslinked polymer hydrogel possessing at least three links between the crosslinker region and the functional polymer region. The links between the crosslinker region and the functional polymer region may be a reaction product of at least one electrophilic functional group from one of the precursors, i.e., the crosslinker or the functional polymer, with at least one nucleophilic functional group on the other precursor.

Further, without being limited to a particular range, a biocompatible crosslinked polymer hydrogel may have a molecular weight of from about 5,000 to about 50,000, in embodiments a molecular weight of from about 7,000 to about 40,000, and in other embodiments a molecular weight of from about 10,000 to about 20,000. The term polymer, as used herein, includes a molecule formed of at least three repeating groups. The term "reactive precursor species" includes a polymer, functional polymer, macromolecule, small molecule, or crosslinker that can take part in a reaction to form a network of crosslinked molecules, e.g., a hydrogel.

### Preparation of Biodegradable Crosslinked Proteins

The biodegradable crosslinkers described in FIGS. 1 and 3 may be reacted with proteins, such as albumin, other serum proteins, or serum concentrates to generate crosslinked polymeric networks. Briefly, aqueous solutions of the crosslinkers described in FIG. 1 and FIG. 3 (at a concentration of from about 50 to about 300 mg/ml) may be mixed with concentrated solutions of albumin (about 600 mg/ml) to produce a crosslinked hydrogel. This reaction can be accelerated if a buffering agent, e.g., borate buffer or triethanol amine, is added during the crosslinking step.

The resulting crosslinked hydrogel is a semisynthetic hydrogel whose degradation depends on the degradable segment in the crosslinker as well as degradation of albumin by enzymes. In the absence of any degradable enzymes, the crosslinked polymer will degrade solely by the hydrolysis of the biodegradable segment. If polyglycolate is used as the biodegradable segment, the crosslinked polymer will degrade in 1-30 days depending on the crosslinking density of the network. Similarly, a polycaprolactone based crosslinked network will degrade in 1-8 months. The degradation time generally varies according to the type of degradable segment used, in the following order: polyglycolate < polylactate < polytrimethylene carbonate < polycaprolactone. Thus it is possible to construct a hydrogel with a desired degradation profile, from a few days to months, using a proper degradable segment.

The hydrophobicity generated by biodegradable blocks such as oligohydroxy acid blocks or the hydrophobicity of PPO blocks in PLURONIC or TETRONIC polymers may be helpful in dissolving small organic drug molecules. Other properties which may be affected by incorporation of biodegradable or hydrophobic blocks include water absorption, mechanical properties, and thermosensitivity.

### Methods of Using Biocompatible Polymers

The biocompatible crosslinked polymers and their precursors described above may be used in a variety of applications, including those disclosed in U.S. Patent Nos. 7,332,566 and 6,566,406, the entire disclosures of each of which are incorporated by reference herein. In embodiments, the compositions of the present disclosure may be utilized for the removal of polyps from an animal.

Some of these approaches allow for the polymers to be added to the patient "in situ" in a solution and then chemically reacted inside the patient so that the polymers form covalent crosslinks to create a polymer network. The in situ approach lets the polymer be formed in a way that closely conforms to the shape of the tissues in the body, as described, for example, in United States patents 5,410,016; 5,573,934 and 5,626,863.

In many applications, the biocompatible crosslinked polymers may be formed "in situ" at a surgical site in the body. The various methodologies and devices for performing "in situ" gelation, developed for other adhesive or sealant systems such fibrin glue or sealant applications, may generally be suitably used with the biocompatible crosslinked polymers. Thus, in one embodiment, an aqueous solution of a freshly prepared crosslinker (e.g., SNHS-terminated oligolactide synthesized from a glycerol core in phosphate buffered saline ("PBS") at pH 5 to 7.2) and a functional polymer (e.g., albumin or amine terminated tetrafunctional polyethylene glycol at pH 10 in sodium borate) may be applied and mixed on the tissue using a double barrel syringe (one syringe for each solution). The two solutions may be applied simultaneously or sequentially. In some embodiments, it may be desirable to apply the precursor solutions sequentially so as to "prime" the tissue, resulting in improved adherence of the biocompatible crosslinked polymer to the tissue. Where the tissue is primed, the crosslinker precursor may be applied to the tissue first, followed by the functional polymer solution.

The in situ formation process may be used to make, for example, coatings, adhesion prevention barriers, tissue glues, matrices for drug delivery, wound dressings, implants, and tissue engineering matrices. In some embodiments, compositions may be introduced into a polyp or the submucosa adjacent a polyp to facilitate removal of the polyp. One may use specialized devices to apply the precursor solutions, including those described in U.S. Patent Nos. 4,874,368; 4,631,055; 4,735,616; 4,359,049; 4,978,336; 5,116,315; 4,902,281; 4,932,942; Published Patent Cooperation Treaty Patent Application No. WO 91/09641; and R.A. Tange, "Fibrin Sealant" in Operative Medicine: Otolaryngology, volume 1 (1986), the entire disclosures of each of which are incorporated by reference herein, to the extent they do not contradict what is explicitly disclosed.

As noted above, in embodiments the compositions of the present disclosure may be utilized as an aid for the removal of a polyp. Preconditioning polyps by injecting these compositions and/or precursors into the polyp or the submucosa of one or more polyps may enhance the benefits of polypectomy, for example, by raising the polyps with a composition that gels or becomes more viscous upon introduction into a patient's body, and/or does not readily escape polyps if the submucosa is breached during snaring or in the course of a piecemeal polypectomy procedure. Such preconditioning may further improve the presentation of a polyp making it easier to grab and/or snare during excision.

In addition, treatment regimens in accordance with the present disclosure may improve a passage blocked by one or more polyps, and/or facilitate the removal of tissue having a propensity to develop into a cancerous lesion. Compositions utilized to raise polyps from the mucosal surface may be injected into the submucosa of one or more polyps and allowed to crosslink in situ thereby forming a gel. In other embodiments the functionalized polymer and crosslinker may be introduced into the submucosa of one or more polyps as described above, whereby they crosslink in situ to form a composition of the present disclosure. The formation of a composition of the present disclosure within the polyp may elevate the polyp away from the tissue possessing the polyp, for example, in some embodiments the colonic wall. Once the polyp is elevated away from the tissue, the polyp may be removed utilizing any method within the purview of those skilled in the art. In some embodiments, a snare may be utilized to remove the polyp. Depending upon the size of the polyp and its location, in some embodiments it may be desirable to remove the polyp piecemeal with a snare. As portions are removed from the polyp, the composition of the present disclosure, which is a hydrogel, does not escape the remaining portions of the polyp and the remaining portions of the polyp remain enlarged and elevated from the tissue surface. Treatment may then continue by removing the one or more polyps while the composition in accordance with the present disclosure remains substantially in the submucosa and to some extent in the polyp. The use of visualization agents in the compositions may be utilized to confirm retention of the composition in the polyp and/or the submucosa.

### Delivery of Compositions

In accordance with the present disclosure, any delivery device within the purview of those skilled in the art may be utilized to introduce biocompatible crosslinked polymers of the present disclosure and/or their precursors into a polyp. In embodiments, a cannula or multi-lumen cannula may be utilized.

The lumens may house and permit the transit of any suitable items and/or devices including, but not limited to, one or more medicines, drugs, blood, medical devices, guide wires, snares suitable for use in polypectomy procedures, electrocautery devices, needles, optical fibers, fiber optic imaging devices, fiber optic diagnostic probes, combinations thereof, and the like. As noted above, in some embodiments the compositions of the present disclosure may be formed in situ, so one lumen may be utilized to introduce the functionalized polymer described above, while a second lumen may be utilized to introduce the crosslinker described above.

In embodiments, a suitable multi-lumen cannula may be a double lumen cannula. A double lumen cannula may possess any configuration within the purview of those skilled in the art. For example, in some embodiments, a single tube with a horizontal division of the tube which places the lumens of the cannula in immediate juxtaposition may be utilized.

In other embodiments, a coaxial double lumen cannula may be utilized. Such a cannula may possess concentric lumens, disposed one within the other.

A cannula in accordance with the present disclosure may be of any suitable length; in embodiments from about 1 meter to about 2.5 meters long, in other embodiments from about 1.25 meters to about 2.3 meters long.

A cannula may be introduced into a patient's body through a conventional colonoscope. While a cannula of the present disclosure may be constructed of any material within the purview of those skilled in the art, in embodiments a cannula of the present disclosure may be constructed of a comparatively soft medical grade plastic or metals such as stainless steel, titanium, and the like. Specific synthetic materials which may be utilized include, but are not limited to, fluoropolymers including polytetrafluoroethylene, polyurethane, polyethylene, polypropylene, high density polyethylene, nylons, polyethylene terephthalate, silicones, combinations thereof, and the like.

In embodiments, a needle may be attached to an end of one lumen of a cannula to facilitate introduction of the functionalized polymer, the crosslinker, or the composition of the present disclosure that has not yet crosslinked, into the mucosal wall or into a polyp during a polypectomy.

As noted above, in embodiments the cannulas may be utilized to introduce a composition for use in endoscopic polypectomy. The compositions may be applied to the submucosa of one or more polyps to improve presentation of the polyp, and also make the polyp easier to capture with an endoscopic instrument such as a snare. For example, compositions of the present disclosure and/or their precursors may be injected into the submucosa of a polyp to improve its presentation.

The various constituents of compositions utilized to raise a polyp from the mucosal surface in accordance with the present disclosure may be combined with numerous ingredients to form products to be applied to the polyp, or other tissues of humans or other mammals. Such products may include a pharmaceutically acceptable carrier or diluent, vehicle or medium, for example, a carrier, vehicle or medium that is compatible with the tissues to which they will be applied. The term "dermatologically or pharmaceutically acceptable," as used herein, means that the compositions or constituents thereof may be suitable for use in contact with tissues or for use in patients in general without undue toxicity, incompatibility, instability, allergic response, and the like.

As noted above, compositions of the present disclosure may be injected into the submucosa of a polyp in amounts sufficient to treat the affected area. As used herein the word "treat," "treating" or "treatment" refers to using active ingredients and/or compositions of the present disclosure, optionally in combination with active ingredients, prophylactically to prevent outbreaks of any undesirable conditions, or therapeutically to ameliorate an existing undesirable condition. A number of different treatments may be now possible, which reduce and/or eliminate undesirable conditions.

As used herein "undesirable condition" refers to any detectable tissue manifestations caused by a polyp or removal thereof. Such manifestations can appear due to a number of factors such as, for example, trauma and/or other diseased or dysfunctional state. Non-limiting examples of such manifestations include the development of bleeding, cancer, inflammation, flakiness and/or other forms of tissue abnormality, and combinations thereof. It is understood, that the listed undesirable conditions are non-limiting and that only a portion of the conditions suitable for treatment in accordance with the present disclosure are listed herein.

As noted above, in embodiments, compositions introduced into the submucosa of a polyp and/or a polyp may contain one or more active ingredients in an effective amount to improve undesirable conditions. As used herein "active ingredients" include, but are not limited to, enzymes such as thrombin, vasoconstrictors such as epinephrine, norepinephrine, angiotensin, or vasopressin, chemotherapeutic agents such as fluorouracil (5-FU), and combinations of these active agents. As used herein, an "effective amount" refers to an amount of a compound or composition having amounts sufficient to induce a particular positive benefit to the polyp or tissue adjacent thereto. The positive benefit can be health-related. In embodiments, the positive benefit may be achieved by contacting tissue with a coagulation protein to promote clotting and closure of the excised tissue. In other embodiments, the positive benefit may be achieved by contacting tissue with a vasoconstrictor to reduce bleeding. In yet other embodiments, the positive benefit may be achieved by contacting tissue with a chemotherapeutic agent to kill cancerous cells.

In order that those skilled in the art may be better able to practice the compositions and methods described herein, the following examples are given as an illustration of the preparation of the present compositions and methods of use thereof. It should be noted that the disclosure is not limited to the specific details embodied in the examples.

### EXAMPLES

The following non-limiting examples are intended to illustrate the synthesis of new biocompatible crosslinked polymers and their precursors, and their use in making several medical products. Those skilled in the art will appreciate that modifications can be made to these examples, drawings, illustrations and claims that are intended to fall within the scope of the present disclosure.

### Materials and Equipment

Polyethylene glycol was purchased from various sources such as Shearwater Polymers, Union Carbide, Fluka and Polysciences. Multifunctional hydroxyl and amine terminated polyethylene glycol were purchased from Shearwater Polymers, Dow Chemicals and Texaco. PLURONIC® and TETRONIC® series polyols were purchased from BASF Corporation. DL-lactide, glycolide, caprolactone and trimethylene carbonate was obtained from commercial sources like Purac, DuPont, Polysciences, Aldrich, Fluka, Medisorb, Wako and Boehringer Ingelheim. N-hydroxysulfosuccinimide was purchased from Pierce. All other reagents, solvents were of reagent grade and were purchased from commercial sources such as Polysciences, Fluka, Aldrich and Sigma. Most of the reagents and solvents were purified and dried using standard laboratory procedures such as described in D.D. Perrin et al., Purification of Laboratory Chemicals (Pergamon Press 1980).

### General Analysis

The polymers synthesized according to these examples were chemically analyzed using structure-determining methods such as nuclear (proton and carbon-13) magnetic resonance spectroscopy, infrared spectroscopy. Molecular weights were determined using high pressure liquid chromatography and gel permeation chromatography. Thermal characterization of the polymers, including melting point and glass transition temperatures, were performed using differential scanning calorimetric analysis. Aqueous solution properties such as micelle and gel formation was determined using fluorescence spectroscopy, UV-visible spectroscopy and laser light scattering instruments.

In vitro degradation of the polymers was followed gravimetrically at 37°C, in an aqueous buffered medium such as phosphate buffered saline (at pH 7.2). In vivo biocompatibility and degradation life times was assessed by injecting or forming a gelling formulation directly into the peritoneal cavity of a rat or rabbit and observing its degradation over a period of 2 days to 12 months.

Alternatively, the degradation was also assessed by prefabricating a sterile implant, made by a process like solution casting, then surgically implanting the implant within an animal body. The degradation of the implant over time was monitored gravimetrically or by chemical analysis. The biocompatibility of the implant was assessed by standard histological techniques.

Some aspects of methods and procedures for certain embodiments set forth herein are provided and set forth in detail in commonly owned U.S. Patent No. 7,009,034, the entire disclosure of which is hereby incorporated by reference herein, to the extent it does not contradict what is explicitly disclosed. Some of these methods and procedures include the synthesis of a water-soluble difunctional, biodegradable functional polymer based on polyalkylene oxide block copolymer, synthesis of amine terminated synthetic biodegradable crosslinkable polymers, synthesis of carboxyl terminated oligolactic acid polymer activated with N-hydroxysulfosuccinimide, preparation of polyethylene glycol based tetrafunctional crosslinker, synthesis of sulfonyl chloride activated crosslinkers, synthesis of multifunctional oligopolycaprolactone, preparation of polyethylene glycol-co-polytrimethylene carbonate copolymer terminated with N-hydroxysuccinimide, synthesis of succinated polyhydroxy compounds activated with N-hydroxysulfosuccinimide ES, preparation of composite synthetic crosslinked colored biodegradable gels, evaluation of the stability of colorants in solution, concentrations of coloring agent for use in an in situ crosslinked hydrogel coating, the effect of coloring agents on gelation times.

Example 1 Preparation of synthetic crosslinked biodegradable gels.

1.57 g (0.8 mM) of 4 arm amine terminated polyethylene glycol molecular weight 2000 was dissolved in 10 ml 0.1 M sodium borate buffer at pH 9.5 2 g of 4 arm succinimidyl ester activated polymer (4PEG2KGS, molecular weight 2500) was dissolved in phosphate buffered saline. These two solutions were mixed to produce a crosslinked gel. In another variation of this method, the 4PEG2KGS polymer solid was directly added to the amine terminated polymer solution to produce a crosslinked polymer.
In another variation, a crosslinker consisting of an equimolar solution of dilysine can be used in place of the 4 arm PEG amine solution to form a hydrogel. Gelation was seen to occur within 10 seconds of mixing the two solutions. The amine terminated polymer solution described above was added with 0.1 % of F D and C blue or indigo dye prior to crosslinking reaction. The addition of dye allows the preparation of colored gels.

Example 2 Formulation of SG-PEG with Di-lysine.

A four arm PEG with succinimidyl glutarate (SG) end groups (Shearwater Polymers, approx. 9,100 g/mol, 0.704 grams, 6.5x10⁻⁵ moles) was dissolved in 2.96 g 0.01 M pH 4.0 phosphate buffer (19.2% solids). Di-lysine (Sigma, 347.3 g/mol, 0.03 grams, 8.7x10⁻⁵ moles) was dissolved in 3.64 grams of 0.1 M pH 9.5 borate buffer (0.8% solids). On combination of the two solutions, a hydrogel formed having a percent solids of 10%. The di-lysine has 3 amine groups. The SG-PEG has 4 NHS groups. After correction for the less than 100% degree of substitution on the SG-PEG, the formulation gives a 1:1 stoichiometry of amine groups to NHS groups.

Example 3 Formulation of SG-PEG with Tri-lysine.

A four arm PEG with SG end groups (Shearwater Polymers, approx. 9,100 g/mol, 0.675 grams, 6.2x10⁻⁵ moles) was dissolved in 2.82 g 0.01 M pH 4.0 phosphate buffer (19.3% solids). Tri-lysine (Sigma, 402.5 g/mol, 0.025 grams, 6.2x10⁻⁵ moles) was dissolved in 3.47 grams of 0.1 M pH 9.5 borate buffer (0.7% solids). On combination of the two solutions, a hydrogel formed having a percent solids of 10%. The tri-lysine has 4 amine groups. The SG-PEG has 4 NHS groups. After correction for the less than 100% degree of substitution on the SG-PEG, the formulation gives a 1:1 stoichiometry of amine groups to NHS groups.

Example 4 Formulation of SG-PEG with Tetra-lysine.

A four arm PEG with SG end groups (Shearwater Polymers, approx. 9,100 g/mol, 0.640 grams, 5.9x10⁻⁵ moles, also referred to herein as being about 10,000 MW) was dissolved in 2.68 g 0.01 M pH 4.0 phosphate buffer (19.2% solids). Tetra-lysine (Sigma, 530.7 g/mol, 0.025 grams, 4.7xIO-' moles) was dissolved in 3.30 grams of 0.1M pH 9.5 borate buffer (0.8% solids). On combination of the two solutions, a hydrogel formed having a percent solids of 10%. The tetra-lysine has 5 amine groups. The SG-PEG has 4 NHS groups. After correction for the less than 100% degree of substitution on the SG-PEG, the formulation gives a 1:1 stoichiometry of amine groups to NHS groups.

Example 5 Gel Time Measurement.

The amine solution (100 µL) was aliquotted into a 100x13 test tube. A flea-stirbar (7x2 mm, Fisher Scientific p/n 58948-976) was placed in the test tube. The test tube was held stationary over a digital magnetic stirrer (VWR Series 400S Stirrer) set at 300 rpm. A 1 cc tuberculin syringe (Becton Dickinson, p/n BD309602) was filled with 100 µL of the ester solution. The syringe was inserted up to the flanges so that the distal end was just over the amine solution. Simultaneously the plunger was depressed and a stop watch started. When the solution solidifies sufficiently so that the stir bar stops spinning, the stop watch was stopped. Each solution was measured in triplicate and the mean ±1 standard deviation was plotted. Results for the formulations of examples 2, 3 and 4 are shown in FIG. 7.

Example 6 Change in gel time as a function of ester solution age.

An important characteristic of these systems is the loss in reactivity over time from reconstitution of the ester solution, also referred to as the ester's pot life. This loss in , reactivity occurs due to hydrolysis of the N-hydroxysuccinimidyl ester, before the activated molecule can combine with its respective nucleophilic functional group. The loss of reactivity was characterized by measuring the change in gel time as a function of time from reconstitution of the NHS ester solution. The gel time was measured periodically. The NHS ester solution was stored at ambient conditions during this measurement. Results for the solutions described in Examples 2, 3 and 4 are shown in FIG. 8.

Example 7 Gel formation at different percent solids from 4 arm CM-HBA-NHS PEG and Lys-Lys.

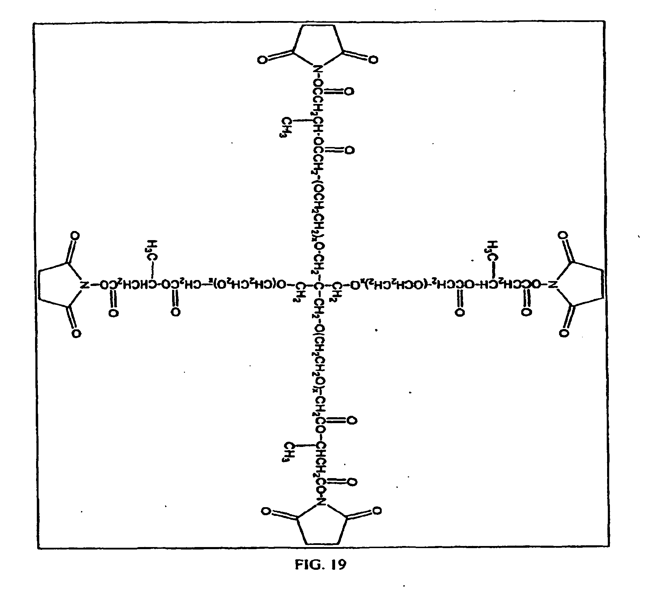

Using the gel time method described in Example 5, five different gel compositions were made using 4 arm PEG (CM-HBA-NHS, see Figure 19, about 10,000 MW) (Shearwater Polymers) and di-lysine (Sigma). The formulations are listed below in Table 3.

**Table 3**

| Conc. (%) | CM-HBA-NHS (g) | Phosphate (g) | Lys-Lys (g) | Borate (g) |
|---|---|---|---|---|
| 8.5 | 0.2469 | 1.264 | 0.01 | 1.5012 |
| 10 | 0.2904 | 1.2209 | 0.012 | 1.4994 |
| 12.5 | 0.363 | 1.1483 | 0.015 | 1.4964 |
| 15 | 0.4356 | 1.0757 | 0.018 | 1.4936 |
| 20 | 0.5808 | 0.9305 | 0.024 | 1.4876 |

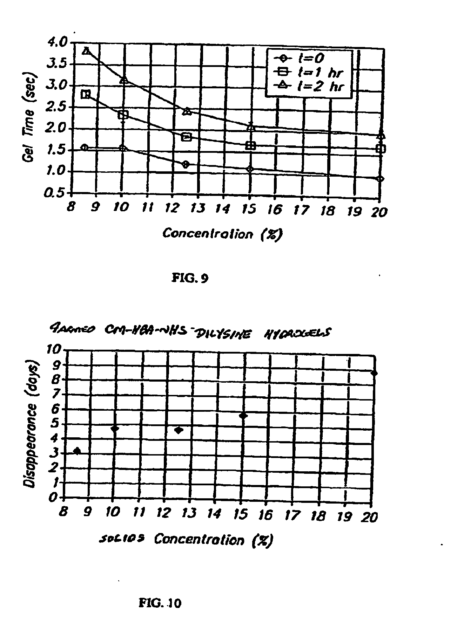

The formulations were adjusted to give a 1 to 1 ratio of electrophilic functional end groups on the CM-HBA-NHS to nucleophilic reactive groups on the di-lysine ("Lys-Lys")(3). The CM-HBA-NHS quantities were dissolved in 0.01 M pH 5.0 phosphate buffer. The di-lysine was dissolved in 0.1 M pH 11 borate buffer. Gel time results are shown in Figure 9. This data also shows that the higher percent solids solutions also are the most stable with respect to retention of speed of reaction.

Example 8 Degradation of Hydrogels.

Hydrogel plugs made as per Example 7 were placed in approximately 25 mL 0.1M phosphate buffered saline at pH 7.4 in 50 mL Falcon tubes and placed in a constant temperature bath at 37°C. The hydrogel plugs were observed visually at periodic intervals and the time of gel disappearance noted. The data are plotted in Figure 10.

Example 9 Precursor Spray Procedure to form a 7.5% solids hydrogel from 4 arm SG and dilysine.

An ethylene oxide sterilized air assisted sprayer was used in conjunction with aqueous solutions of polymerizable monomers. Solution 1 consisted of a 14.4% solution of 4 arm PEG succinimidyl glutarate (SG-PEG, MW 10,000 purchased from Shearwater Polymers) dissolved in 0.01 M phosphate buffer at pH 4.0 and was sterile filtered (Pall Gelman syringe filter, p/n 4905) and drawn up in a sterile 5 cc syringe. Solution 2 consisted of a 1.2% solution of a dilysine (purchased from Sigma Chemicals) dissolved in 0.1 M borate buffer at pH 11 with 0.5 mg/mL methylene blue for visualization and was also sterile filtered and drawn up in a sterile 5 cc syringe. These solutions, when combined 1:1 on a volumetric basis, resulted in a 1:1 ratio of NHS ester to amine end group. The final % solids after combination was 7.5%. The two syringes were individually loaded in the two separate receptacles through a LUER-LOK type of linkage. Airflow from a regulated source of compressed air (an air compressor such as those commercially available for airbrushes) was connected to the device using a piece of TYGON tube. On compressing the syringe plungers a steady spray of the two liquid components was observed. When this spray was directed to a piece of tissue (rat cecum) a hydrogel coating was observed to form on the surface of the tissue. This hydrogel coating was rinsed with saline (the hydrogel coating is resistant to rinsing) and was observed to be well adherent to the tissue surface. Within a short period of time (less than a minute) an area of 10 cm X 5 cm could be coated with ease.

Example 10 Precursor Spray Procedure to form a 12.5% solids hydrogel from 4 arm CM-HBA-NHS and dilysine.

A hydrogel barrier film made from 4 arm CM-HBA NS (MW 10,000 purchased from Shearwater Polymers), and dilysine was similarly prepared and sprayed as described in Example 9. In the present example the 4 arm CM-HBA-NHS solution was made up to 24.0% solids and the dilysine solution was made up to 1.0% solids such that on combination in an equal volume delivery system a 1:1 ratio of NHS to amine end groups results, giving a final % solids of 12.5%. This formulation was effective for making a hydrogel.

Example 11 Spray Application of crosslinker and polymer to form crosslinked film.

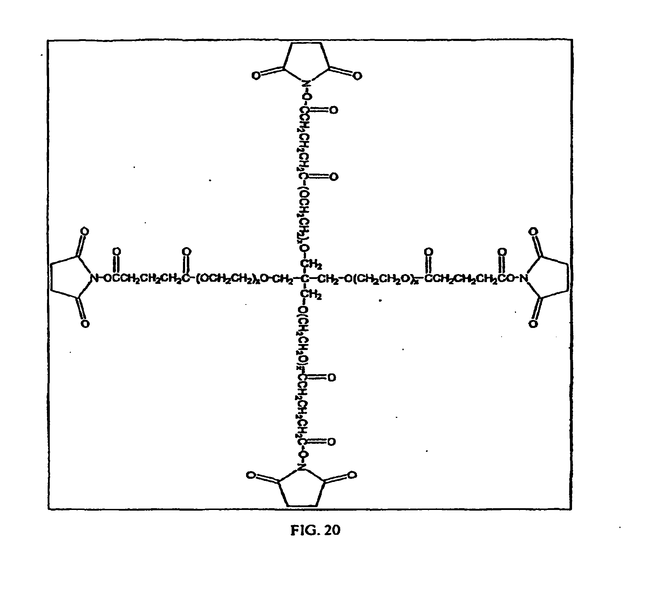

Two solutions (component A and component B) were prepared. Component A consisted of dilysine in 0.1 M borate buffer, pH 9.5. Component B consisted of either 4 arm SG-PEG (Figure 20) or 4 arm CM-HBA-NHS (Figure 19) in 0.01 M phosphate buffer, pH 4.0 These solutions were prepared such that the amine to ester stoichiometric ratio was 1:1 and the final total solution concentration was 7.5% or 12.5%, respectively.

A FIBRIJECT (Micromedics, Inc.) 5 cc syringe holder and cap was used, preloaded with 5 cc of each solution and attached to a dual barrel atomizing sprayer. The sprayer has two hubs for the syringes to connect to allowing the two fluids to be advanced through two separate lumens over any preset distance. A third hub exists for the application of the atomizing gas. Air was used in this example. The distal tip of the sprayer contains a chamber where the gas expands out of an introduction tube, then flows past the two polymer solution nozzles in an annular space around each. The gas is accelerated in the annular spaces using a flow rate suitable for the complete atomization of the two fluid streams (∼2L/min.). Two overlapping spray cones are thus formed allowing for well mixed, thin, uniform coatings to be applied to surfaces, and such coatings resulted from mixtures of A and B.

Example 12 Adhesion Prevention in Rat Cecum Model.

Surgical procedure. Male Sprague Dawley rats (250-300 grams,) were anesthetized with an intramuscular 4ml/kg "cocktail" of Ketamine (25 mg/ml), Xylazine (1.3mg/mL) and Acepromazine (0.33 mg/mL). The abdominal area was shaved and prepped for aseptic surgery. A midline incision was made to expose the abdominal contents. The cecum was identified and location within the abdomen was noted. The cecum was pulled out of the abdomen and the surface of one side was abraded using dry sterile gauze. A technique of abrading one area by stroking the surface 12 times with the gauze was used. The cecal arterial supply was interrupted using bipolar coagulation along the entire surface area of the damaged cecum.

The opposing abdominal sidewall which lays in proximity to the damaged cecal surface was deperitonealized with a scalpel blade and the underlying muscle layer was scraped to the point of hemorrhaging.

The cecum was sprayed with either the SG-PEG system or the CM-HBA-NHS system using the air assisted spray method described in the preceding example. The cecum was placed with the damaged (ischemic area) side up opposite the damaged side wall. Active bleeding was controlled before closing. The peritoneum and muscle wall was closed with 3-0 nylon and the skin was closed with 4-0 silk. Rats were returned to their cages for one to two weeks at which time evaluation of the adhesion between the side wall and cecum was noted. The rats were killed at 10 days and the tenacity and extent of adhesion was evaluated. The results are summarized in Table 4, with 4a SG standing for SG-PEG as shown in Figure 20, MB standing for methylene blue, and 4a CM standing for CM-HBA-NHS as shown in Figure 19.

**Table 4**

| Rat # | Material Applied | Reference Example | Finding on Day 10 |
|---|---|---|---|
| 403 | 7.5% 4aSG with Lys-Lys w/MB | Example 9 | Small amount of gel present on cecum. No adhesions from cecum to sidewall. No gel on sidewall |
| 404 | 7.5% 4aSG with Lys-Lys w/MB | Example 9 | Some mesentery stuck to cecum. No gel. No adhesions. |
| 405 | 7.5% 4aSG with Lys-Lys w/MB | Example 9 | Small amount of gel present on cecum. Some mesentery stuck to cecum and sidewall. Some gel between mesentery and cecum where stuck. No adhesions. |
| 406 | 12.5% 4aCM with Lys-Lys w/MB | Example 10 | No gel present. No adhesions. |
| 407 | 12.5% 4aCM with Lys-Lys w/MB | Example 10 | No gel on cecum or sidewall. No adhesions. |
| 408 | 12.5% 4aCM with Lys-Lys w/MB | Example 10 | Rat died post-op (anesthesia overdose). |

Example 13 Control of degradation time of hydrogels using mixtures of low molecular weight molecules of trilysine and Tris(2-aminoethyl)amine (Tris).

This example shows that the swelling, gelation time, and degradation time of a hydrogel is controllable by incorporating various amounts, types, and combinations of low molecular weight amines in the hydrogel. The low molecular weight precursors were nucleophiles of trilysine (Bachem, see Figure 11) or Tris (Aldrich, see Figure 12) that were reacted with a 4-armed PEG-SG-NHS with MW of about 20,000 (Figure 20). Gel time, swelling, and degradation of the hydrogels were measured as a function of the relative amounts of Tris and trilysine. All hydrogel formulations were made with 9.1% solids, in pH 9.5 buffers with a 1 to 1 ratio of nucleophiles to electrophiles. The mixing was accomplished by connecting syringes loaded with the precursor solutions to a mixing tip (ASHBY-CROSS, STATOMIX) that quickly and thoroughly mixed the solutions.

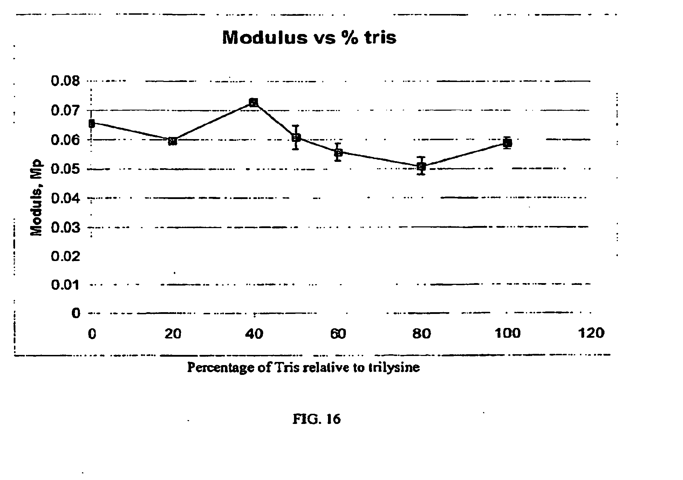

Figures 13, 14, 15, 16 show the results of gelation time, swelling, degradation, and mechanical properties tests, respectively. Increased amounts of Tris relative to the amount of trilysine unexpectedly accelerated gelation, increased swelling, and accelerated degradation rates. Significantly, these trends were approximately linear so that the ratio of nucleophiles could be predicted and chosen to achieve a desired hydrogel property, e.g., a desired degradation rate. Gelation measurements were performed by the methods of Example 5.

Swelling measurements were performed by forming gels of a defined geometry and measuring their weight as a function of time immersed in 37° C phosphate buffered saline (PBS). Weight measurements were performed by weighing the samples before immersion or by removing immersed samples from the PBS, blotting them with a dry towel, and weighing them. The % swelling was defined as ((final weight - initial weight)/initial weight)*100.

The degradation tests were performed by forming gel plugs in a 3 cc syringe, forcing them out of the plunger-end of the syringe with PBS forced through the syringe tip, and placing the plugs in 50 ml centrifuge tubes filled with 20 ml PBS and stored at 37° C and monitored until they were no longer visible to the human eye, a test also referred to herein as measuring the disappearance time of the gels.

Example 14 Degradation of hydrogels made from trilysine.

This example evaluated the persistence time of a hydrogel made from a four armed 20,000 MW PEG-SG-NHS precursor and a trilysine precursor as described in Example 13. The precursors were mixed and gelled in a 3 cc capacity plastic syringe, pushed out of the syringe, and divided into four pieces weighing about 0.25 g each. Two parts were inserted subcutaneously in pockets created by blunt dissection in each rat, one on either side of the midline on the back of the rat. One piece was placed in each pocket. The subcutaneous pockets were closed with interrupted sutures, and the animals were allowed to recover. At scheduled timepoints tissue samples were taken from the subcutaneous pocket and fixed in fomalin for histological evaluation according to methods within the purview of those skilled in these arts. At each timepoint, (4 days, 1 week, 2 weeks, 3 weeks, 4 weeks), the subcutaneous pockets were opened and the gel plugs were evaluated using the following scoring system: 4 = solid gel; 3 = loose, extrudable gel; 2 = viscous liquid; 1 = no gel. The gels were clear at all timepoints and no infections were noted throughout the study. Results are shown in Table 5. Gels appeared to be present at 35 days; no data was recorded after 35 days.

**Table 5**

| Observations of trilysine hydrogels in vivo | | |
|---|---|---|
| Rat Number | Days after implant | Observations |
| 1 | 4 | Score = 4 on both sides. Gels were clear, had no infection |
| 2 | 7 | Score = 4 on both sides. Gels were clear, had no infection |
| 3 | 15 | Score = 4 on both sides. Gels were clear, had no infection |
| 4 | 21 | Score = 4 on both sides. Gels were clear, had no infection |
| 5 | 28 | Score = 3.5 on one side and no gel found on the other side. Gels were clear, had no infection |
| 6 & 7 | 35 | Gels were present as determined by palpitation of the subcutaneous pockets, gels were not removed. |

Example 15 Degradation of hydrogels made from trilysine or Tris.

This example evaluated the persistence time of a hydrogel made from four armed 20,000 MW PEG-SG-NHS and trilysine or Tris. To make a hydrogel including trilysine, about 0.8 g of trilysine (Bachem) was added to 19 ml of 0.1 M borate followed by pH adjustment to 10.5. About 0.4 g of the four armed PEG-SG-NHS (Shearwater) was dissolved in 1.65 ml 0.01 M phosphate buffer, pH 4.0 immediately before use to make a solution of about 18% solids. The solutions were mixed in 1:1 proportions, with a final solids concentration in the hydrogel of about 9%. The mixing was accomplished by connecting syringes loaded with the solutions to a mixing tip (ASHBY-CROSS, STATOMIX) that quickly and thoroughly mixed the solutions.

To make a hydrogel including Tris, about 10 ml of 0.14% Tris (Aldrich) was made by adding about 0.014 g of Tris to 10 ml 0.1 M borate, and the pH was adjusted to pH 8.5. About 0.3 g of the SG-NHS (Shearwater) was dissolved immediately before use in 1.7 ml of phosphate buffer, pH 4.0, so that a solids concentration of about 15% was achieved. The solutions were mixed in 1:1 proportions, with a final solids concentration in the hydrogel of about 8%.

The gels were made in a 3 cc syringe, implanted into rats, and evaluated for degradation as described in Example 14. The results for hydrogels including Tris and trilysine are shown in Figures 17 and 18, respectively. The Tris based hydrogels were completely degraded within 4 to 8 days. The trilysine based gels persisted longer. At about 22 days, the trilysine gels were noticeably degraded and at day 29 the gels were liquefied.

Example 16 Degradation and properties of dilysine-based hydrogels.

This experiment produced data showing degradation profiles for hydrogels made from dilysine plus a four armed carboxymethyl Hydroxybutyrate N-Hydroxysuccinimidyl polyethylene glycol (CM-HBA-NHS, see Fig. 19, "4aCM") of about 10,000 MW or a 20,000 MW PEG-SG-NHS, see Fig. 20, "4aSG". Formulations of hydrogels made from 4 armed 10,000 MW CM-HBA-NHS and eight armed 20,000 MW PEG amine are plotted as "X" in Figures 21 and 22. Gelation time, swelling, and the effect of solids concentrations are also reported, with the tests being performed essentially as described in Example 13.

A nebulizer connected to a sprayer that rapidly combined and thoroughly mixed the component solutions as a spray was used to combine the precursor solutions to create the hydrogels, as described in commonly owned and assigned U.S. patent No. 6,165,201. The nucleophilic precursor solution was made by dissolving the nucleophile (e.g., dilysine) in pH 11.0, 0.1 M buffer. The electrophilic precursor solution was made by dissolving the electrophilic precursor (e.g., 10,000 MW CM-HBA-NHS or 20,000 MW PEG-SG-NHS) in about 2.3 ml of 0.05 M phosphate buffer of pH 5.0. The precursor solutions were combined in a 1:1 v/v ratio to achieve the reported solids concentration. The hydrogel was formed on a horizontal Mylar surface, cut into three pie-shaped samples, and their degradation and swelling properties were measured as described in Example 13.

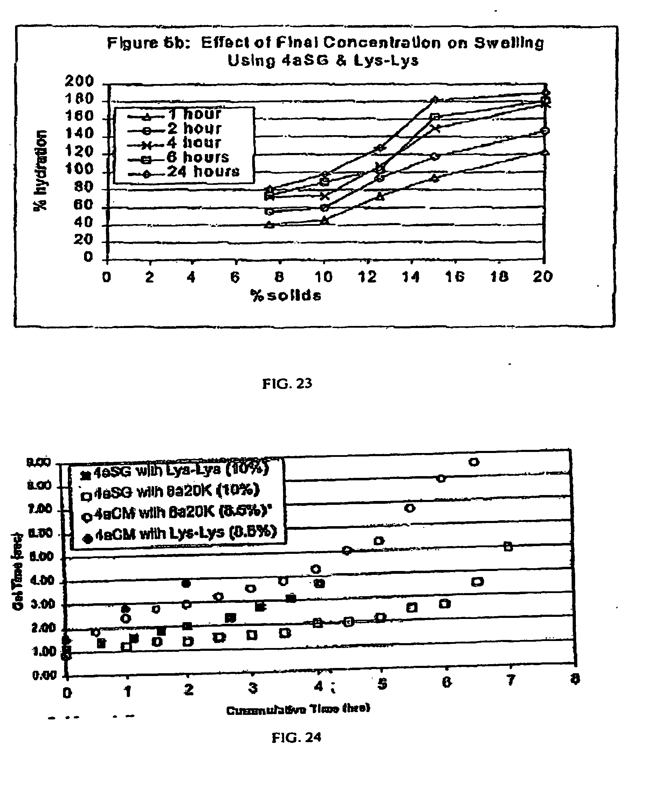

Figures 21, 22, and 23 show the degradation rate, gelation time, and swelling, respectively, of hydrogels formed with dilysines. The time for a gel to disappear (a measure of degradability) increased as the solids concentration increased, with the time increasing faster for hydrogels made with PEG-SG-NHS as compared to CM-HBA-NHS, see Figure 21. The gelation time decreased as the solids concentration increased, see Figure 22. The swellability of the hydrogels increased as the solids concentration increased, see Figure 23.

Other experiments were performed to determine the "pot life" of the formulations, see Figure 24. The pot life is a measure of the time that the chemical activity of a solution will be maintained. In general, NHS-esters lose the ability to covalently bind nucleophiles in aqueous solutions. Thus NHS-esters have a limited pot life in aqueous solutions so that it may be desirable to store them in a dry form and reconstitute them in solution immediately before use. Figure 24 depicts the amount of time between reconstitution and use on the x-axis. The gelation time increased as the storage time of the NHS-esters in the aqueous solution increased. The legend of Figure 24 shows the combinations tested, with 4aSG indicating the four armed PEG-SG-NHS of about 20,000 MW, 4a CM indicating 4 armed 10,000 MW CM-HBA-NHS, Lys-Lys indicating dilysine, 8a20K indicating an eight armed 20,000 MW PEG terminating in primary amines, and the percentages in parentheses indicating solids concentrations in the hydrogels.

Example 17 Degradation and swelling properties of hydrogels made with small molecule precursors.

Hydrogels made of trilysine were compared to hydrogels made with Spermine, Spermidine, Ornithine and Dilysine. Various trilysine formulations were made to demonstrate the wide range of characteristics that could be developed using small molecule precursors, including formulations with varying percent solids and pHs.

This Example produced data showing the properties of hydrogels made with a variety of low molecular weight precursors: Tris, trilysine, ornithine, spermidine, dilysine, and spermine, having structures depicted in Figures 12, 11, 27, 25, 28, and 26, respectively. Hydrogels of these compositions were made essentially as described in Example 13, using four armed 10,000 MW SG-PEG as the electrophile. The solution pH of each nucleophile was further adjusted to pH 10.2, or as indicated. The solids concentration for each hydrogel was 8.5%, or as indicated, and the electrophile: nucleophile stoichiometric ratio was kept constant at 1:1. The formulations were tested for gelation time, swelling, and degradation essentially as described in Example 13.

Other experiments (not described herein) show that the secondary amines of spermine and spermidine essentially do not participate in covalent bonding with the electrophiles so that they have a nucleophilic functionality of 2. Apparently, since spermine and spermidine are long in length compared to the other small molecules tested, they make hydrogels that have properties somewhat different from hydrogels made with trilysine, Tris, dilysine, and ornithine.

When the pH of the formulation solution used to make trilysine hydrogels was increased, gel times and disappearance times decreased while swelling increased. As the percent solids was increased, gel times were faster while swelling and disappearance times increased.

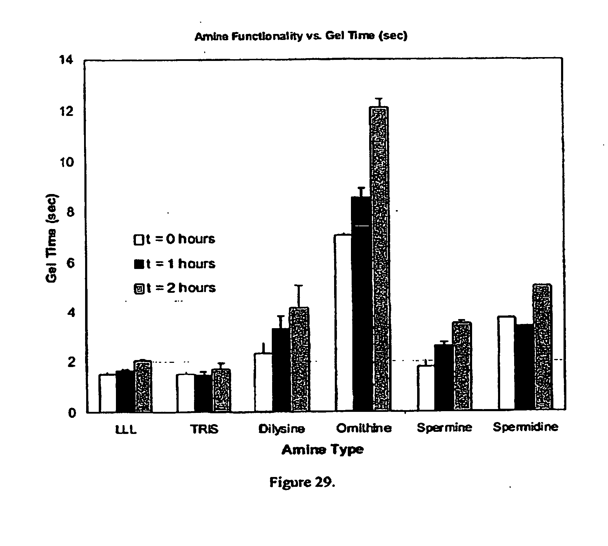

Figure 29 depicts the gelation times for the hydrogels at 1 and 2 hours after reconstitution of the precursors into solution. LLL denotes trilysine. Trilysine has four amines, Tris and dilysine have three amines, and ornithine, spermine, and spermidine have two amines.

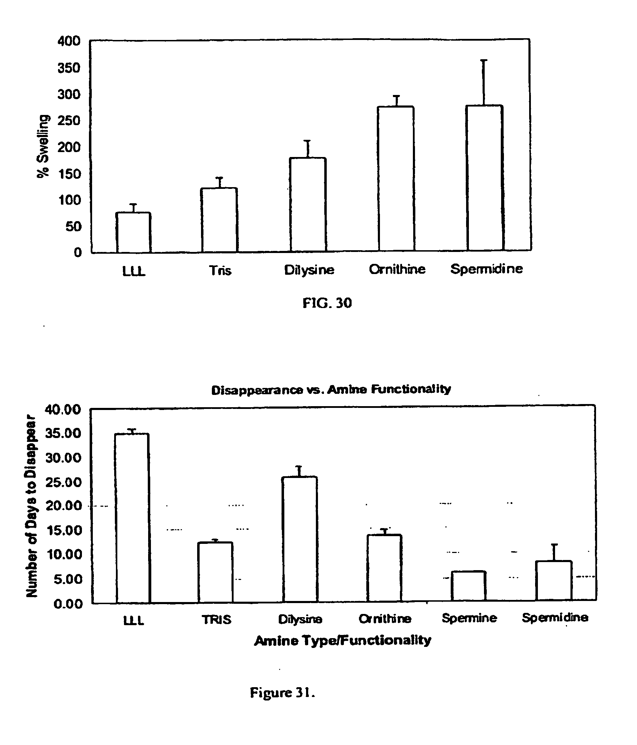

Figure 30 depicts swelling data for the hydrogels made with small molecule precursors. Swelling also showed a trend with functionality of the low molecular weight nucleophilic precursor. As the functionality of the nucleophilic precursor increased, the network was less able to expand. The linear molecules act as chain extenders and do not add to the crosslinking density of the network. The spermine samples at 24 hours were so loose that their swelling could not be measured, apparently due to degradation of the hydrogel.

Figure 31 depicts the degradation of hydrogels made with low molecular weight amines as a function of the number of amines. Generally, hydrogels made with small molecules having a greater amine functionality required longer times to degrade compared to hydrogels made with small molecules having a lower amine functionality, with the exception of hydrogels made with Tris, which degraded more quickly than other hydrogels having three or four amines.

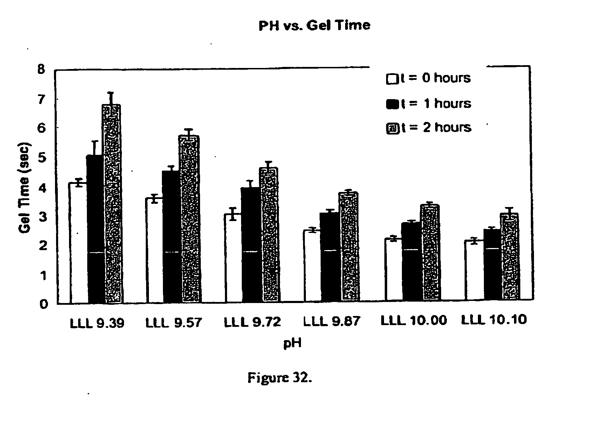

Figure 32 depicts the gelation time for trilysine as a function of the pH of the formulation solution. In general, an increase of the pH of the solution containing the precursors in the range between about 9 and about 10 caused a decrease in gelation time. Also, an increase in the time between reconstitution of the electrophile in aqueous solution and the formation of the hydrogel caused a decrease in gelation time.

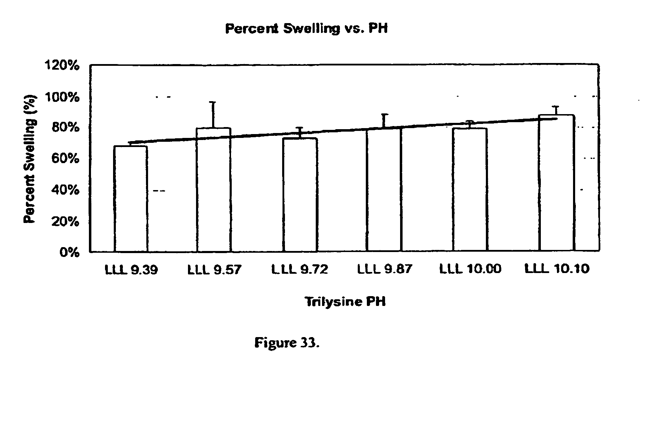

Figure 33 depicts the relationship of swelling as a function of the pH of the formulation solution. As the pH of the formulation solution was increased from about 9 to about 10, the swelling of the resultant hydrogels increased.

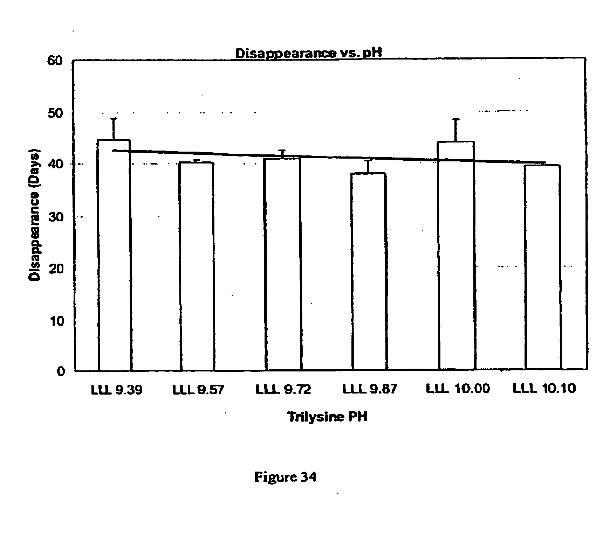

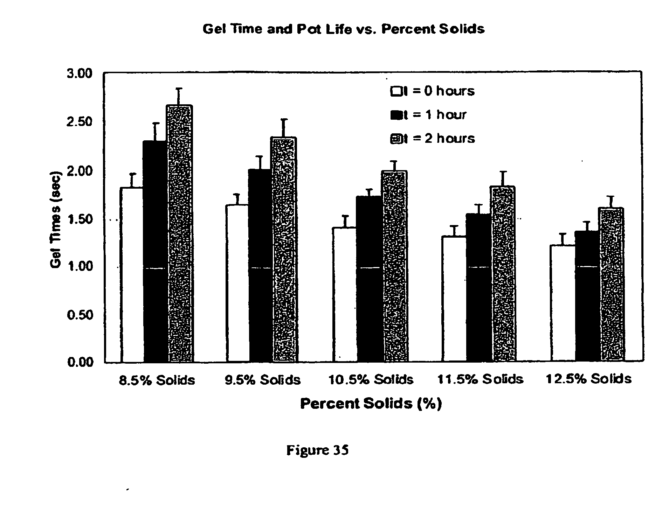

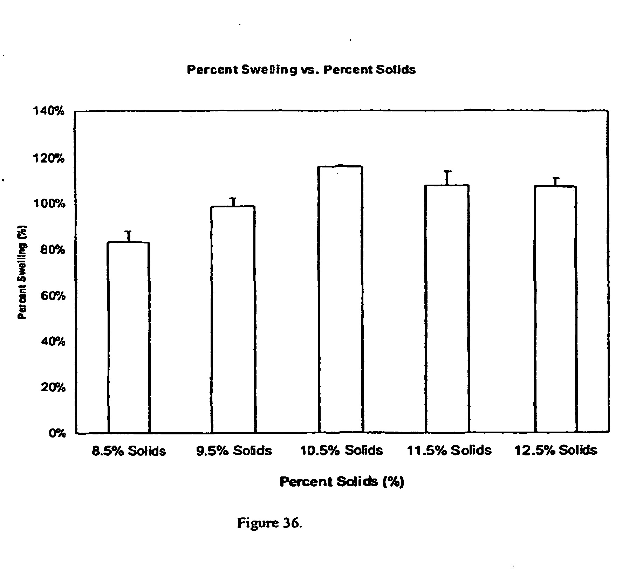

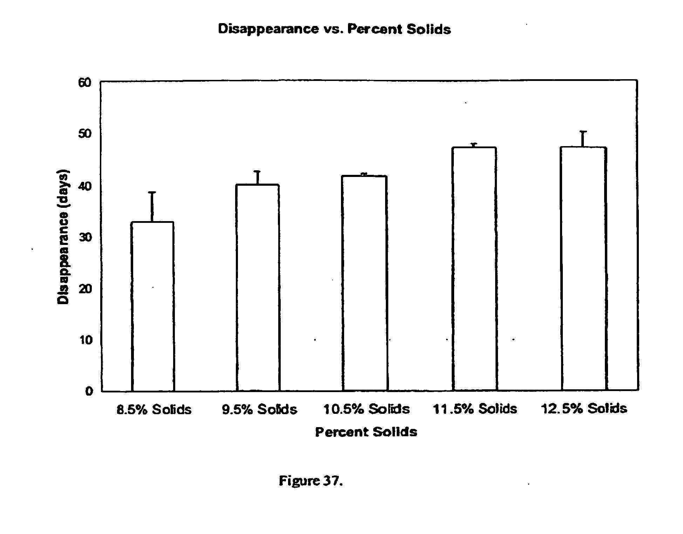

Figure 34 depicts the degradation rate of hydrogels as a function of pH. Figure 35 depicts the gelation time as a function of the total percent solids and the formulation solution pot life. Gelation time decreased as the percent solids in the formulation solution increased from about 8 to about 13 percent. Figure 36 depicts the swelling of hydrogels formed from trilysine as a function of the amount of solids in the formulation solution. Figure 37 depicts the degradation of hydrogels formed from trilysine as a function of the amount of solids in the formulation solution.

Example 18

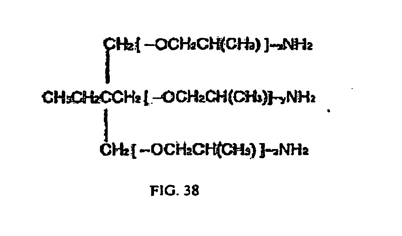

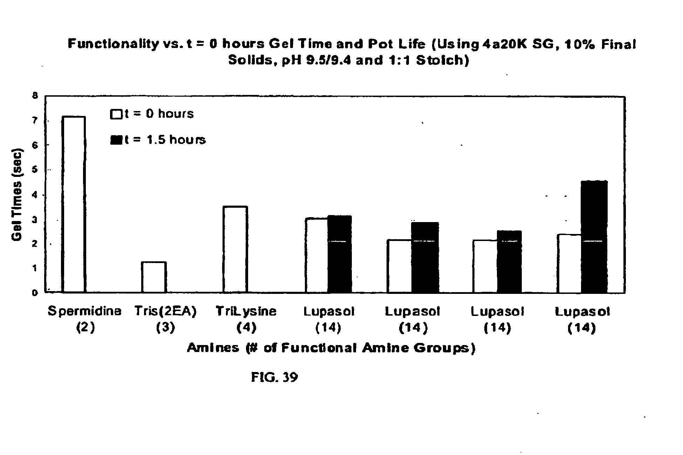

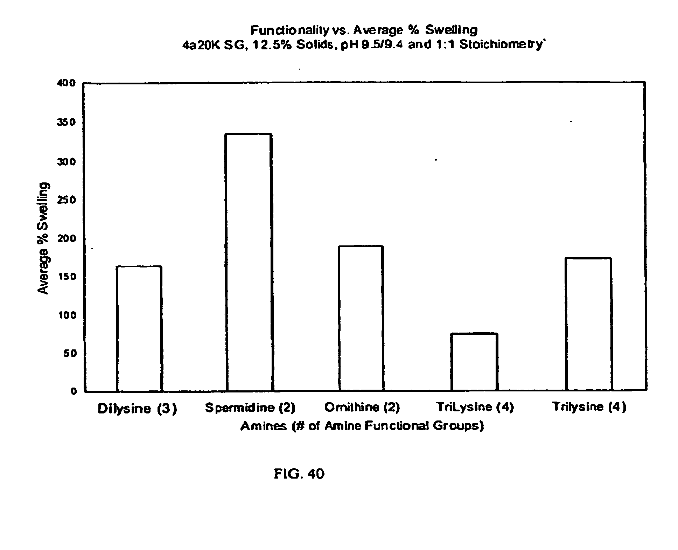

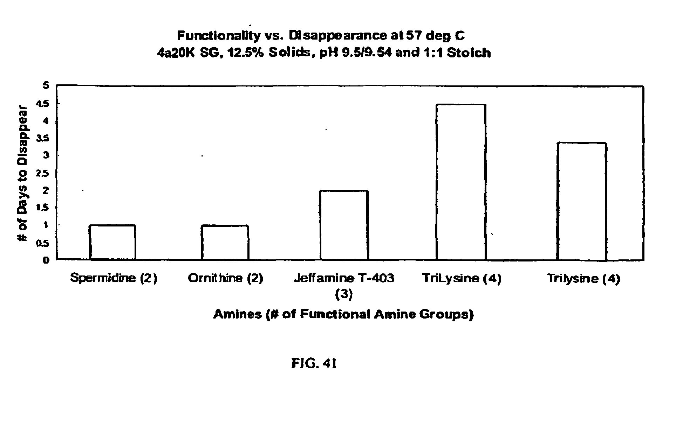

This example shows the results of experiments performed with the nucleophiles spermidine, ornithine, JEFFAMINE T-403 (Figure 38), LUPASOL polyethyleneimine, and trilysine. The electrophile in these experiments was a four-armed 20,000 MW PEG-SG-NHS. Hydrogels of these compositions were gelled and tested for gelation time, swelling, and degradation essentially as described in Example 13. The solution pH of each nucleophile and electrophile was further adjusted to about pH 9.5. The solids concentration for each hydrogel was 12.5%, and the electrophile: nucleophile stoichiometric ratio was kept constant at 1:1. Figures 39, 40, and 41 show the results for the gelation times, swelling, and degradation, respectively. Figure 39 shows the gelation time for hydrogels made immediately following placing the electrophile into solution (t=0) and for 1.5 hours after putting the electrophile into solution (t=1. 5). Figure 41 shows accelerated degradation at 57°C.

Example 19 Methods for using low molecular weight precursors to make hydrogels with predictable degradability.

This example shows methods for choosing formulations to make degradable hydrogels. Included is data that allows the identification of a hydrogel with desired properties of gelation time, water uptake, degradation, and mechanical properties. The electrophile was a 4 armed 20,000 MW PEG-SG-NHS (Shearwater Polymers). The nucleophile was trilysine (Bachem). Hydrogels of these compositions were gelled and tested for gelation time, swelling, and degradation essentially as described in Example 13. The pH, solids concentration, and stoichiometry of nucleophile:electrophile were controlled as indicated. Figures 42, 43, 44, and 45 show the gelation time, swelling, degradation, and modulus, respectively, of the hydrogels, with the ratios indicating nucleophile:electrophile stoichiometric (Stchm.) ratios, and the pH and solids content being as indicated.

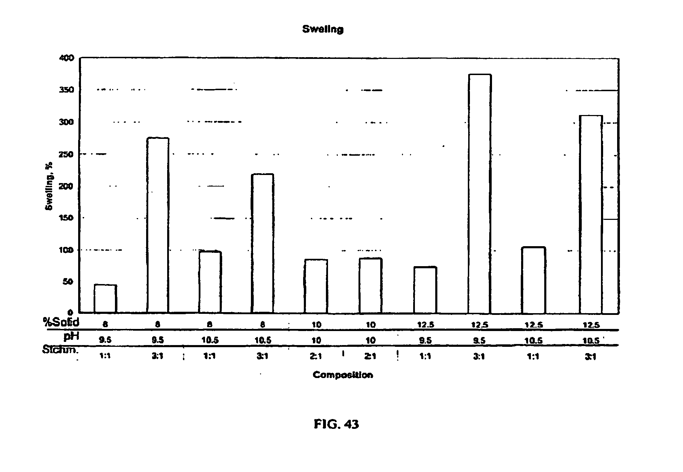

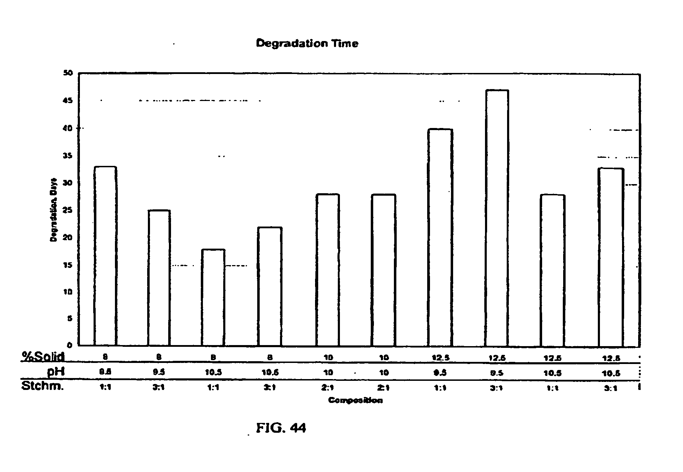

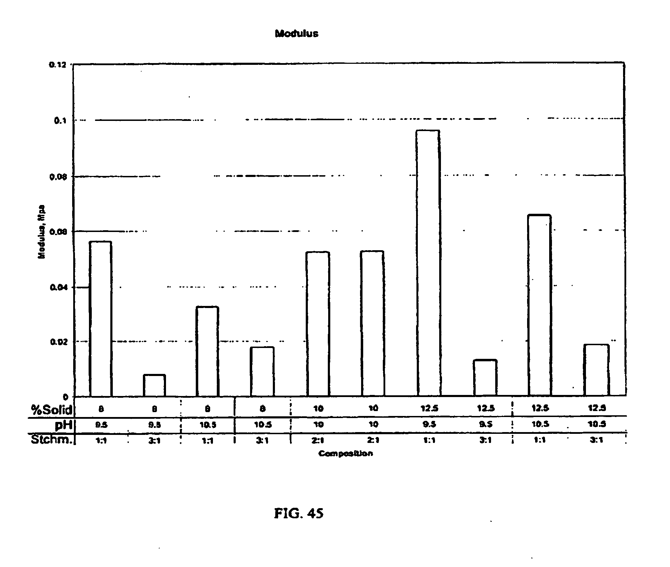

To illustrate the identification process, a user that desired to identify a formulation with a gelation time of less than 2.5 seconds, less than 100% water uptake, an in-vitro degradation time of four weeks or less, and a high modulus would be able to review the indicated Figures and use Figure 43 to eliminate all 3:1 nucleophile to electrophile stoichiometry samples since they would fail the water uptake criterion and because, as demonstrated in Figure 45, they had a lower modulus than the 1:1 ratio hydrogels, which had a modulus of over 30 kPa. The user could refer to Figure 42 to ascertain that 1:1 ratio gels that satisfy the gelation time criterion have solids concentrations between 12.5% and 8.0%. Referring to Figure 44, the degradation rates of a hydrogel can be controlled, in part, by altering the pH of the formulation solutions, so that the pH between more than about 9.5 to about 10.5 would be suitable.

Example 20 Hydrogels made with Dilysine as the low molecular weight precursor.

This example shows the properties of hydrogels made with various formulations of electrophiles in combination with Dilysine. The electrophiles were four armed 10,000 molecular weight PEG-SG (4a10k PEG-SG), four armed 20,000 molecular weight PEG-SG (4a20k PEG-SG), two armed 10,000 molecular weight PEG terminated in N-hydroxy succinimidyl carbonates (2a10k PEG-SC), and eight armed 10,000 molecular weight PEG terminated in N-hydroxy succinimidyl carbonates (8a10k PEG-SC). The hydrogels were made and tested essentially as described in Example 13, with dilysine (ICN Biomedical) being dissolved in 0.1 M pH 10.0 borate buffer and the electrophiles being dissolved in 0.01 M pH 4.0 phosphate buffer.

Table 6 shows the gel times for dilysine precursors reacted at pH 10.0 with various electrophiles mixed to achieve 12.5% solids concentrations. Table 6 shows the effect of the time lapse between reconstitution of the electrophiles of Table 6 and their gelation with dilysine. Table 6 shows the swelling of hydrogels made with various electrophiles reacted with dilysine at pH 10.0 and 12.5% solids, with 4a indicating four arms, 10k indicating a molecular weight of about 10,000.

**Table 6**

| Gel times for dilysine precursors reacted with various electrophilic precursors | |
|---|---|
| Electrophile Formulation | Average Initial Gel Time (seconds) |
| 4a10k PEG-SG | 3.19±0.03 |
| 4a20k PEG-SG | 2.76±0.04 |
| 2a10k PEG-CM-HBA-NHS | No gel formed |
| 8a10k PEG-CM-HBA-NHS | 1.85±0.04 |

**Table 7**

| The effect of the time lapse between reconstitution of the electrophiles of Table 6 from the powder form to aqueous solution and their gelation with dilysine | | |
|---|---|---|
| Electrophile | Cumulative time since reconstitution (hours) | Average Gel Time (Seconds ± Std. Dev.) |
| 4a10k PEG-SG | 0 | 3.19 ±0.03 |
| | 0.5 | 3.75 ± 0.02 |
| | 1.0 | 3.19 ± 0.03 |
| | 1.5 | 5.11 ± 0.04 |
| 4a20k PEG-SG | 0 | 2.76 ±0.04 |
| | 0.5 | 2.96 ± 0.04 |
| | 1.0 | 3.38 ± 0.04 |
| | 1.5 | 3.52 ± 0.04 |
| 8a10k PEG-SC | 0 | 1.85 ±0.04 |
| | 0.5 | 2.68 ± 0.01 |
| | 1.0 | 2.85 ± 0.06 |
| | 1.5 | 3.52 ± 0.05 |

**Table 8**

| comparison of swelling for hydrogels of Table 6 made with various electrophiles and dilysine at pH 10.0 in 12.5% solids | |
|---|---|
| FORMULATION | AVERAGE % SWELLING |
| 4a10k PEG-SG | 70.6 ± 10.8 |
| 4a20k PEG-SG | 165.5 ± 8.8 |
| 2a10k PEG-CM-HBA-NHS | No gel |
| 8a10k PEG-CM-HBA-NHS | 45.1 ± 0.6 |

**Table 9**

| Times to degrade gels of Table 6 in vitro. | |
|---|---|
| FORMULATION | Time (days) |
| 4a10k PEG-SG | 32 |
| 4a20k PEG-SG | 28 |
| 2a10k PEG-CM-HBA-NHS | No gel |
| 8a10k PEG-CM-HBA-NHS | Less than 28 |

Example 21 Physical properties of selected hydrogels.

This Example shows physical properties of hydrogels obtained by mixing certain combinations of electrophiles and nucleophiles. Procedures for making and testing the hydrogels were as per Example 13 unless otherwise indicated. The electrophiles that were tested were four armed 10,000 molecular weight PEG-SG (4a10k PEG-SG), four armed 20,000 molecular weight PEG-SG (4a20k PEG-SG), and 10,000 molecular weight, four armed CM-HBA-NHS PEG (4a10k CM-HBA-NHS). The nucleophile was trilysine (LLL) or 8 armed 20,000 molecular weight PEG amine (8a20k amine). The electrophile: nucleophile ratio was approximately 1:1, the pH of the mixture was 9.5, the electrophiles were reconstituted in pH 4.0 0.01 molar phosphate buffer.

In general, the 4a20k PEG-SG exhibited significantly higher energy to failure than the other two electrophiles. The modulus of the 4a20k PEG-SG was the lowest at 0.058 MPa, compared to 0.086 for the 4a10k CM-HBA-NHS, and 1.121 MPa for the 4a10k PEG-SG.

## Claims

1. A composition comprising a biocompatible small molecule crosslinker with a molecular weight of 2000 or less, the crosslinker having n crosslinker functional groups, wherein n is two or more, and wherein the crosslinker functional groups are either electrophilic or nucleophilic, in combination with a synthetic biocompatible functional polymer with a molecular weight of at least about 7 times more than the crosslinker, the functional polymer having m functional polymer functional groups, wherein m is two or more and the sum of n and m is five or more, and wherein the functional polymer functional groups ame nucleophilic if the crosslinker functional groups are electrophilic, and the functional polymer functional groups are electrophilic if the crosslinker functional groups are nucleophilic for use in a method wherein the method comprises introducing into tissue comprising submucosa of a polyp said composition in combination with said synthetic biocompatible functional polymer and permitting the crosslinker and functional polymer to react in the submucosa of the polyp to form a hydrogel; and
removing the polyp.

2. The composition of claim 1, wherein providing a biocompatible small molecule crosslinker further comprises providing a biocompatible small molecule crosslinker having a solubility of at least 1 g/100 ml in an aqueous solution.

3. The composition of claim 1, wherein providing a biocompatible small molecule crosslinker further comprises providing a biocompatible small molecule crosslinker having crosslinker functional groups that are electrophilic.

4. The composition of claim 3, wherein providing a biocompatible small molecule crosslinker having crosslinker functional groups that are electrophilic further comprises providing a biocompatible small molecule crosslinker wherein the electrophilic crosslinker functional groups are N-hydroxysuccinimide-based crosslinker groups.

5. The composition of claim 3, wherein providing a synthetic biocompatible functional polymer further comprises providing a synthetic biocompatible functional polymer wherein the functional polymer functional groups are amines.

6. The composition of claim 1, wherein providing a biocompatible small molecule crosslinker further comprises providing a biocompatible small molecule crosslinker having crosslinker functional groups that are nucleophilic.

7. The composition of claim 6, wherein providing a biocompatible small molecule crosslinker having crosslinker functional groups that arc nucleophilic further comprises providing a biocompatible small molecule crosslinker wherein the crosslinker functional groups are amines.

8. The composition of claim 6, wherein providing a synthetic biocompatible functional polymer further comprises providing a synthetic biocompatible functional polymer wherein the functional polymer functional groups are N-hydroxysuccinimide groups.

9. The composition of claim 1, wherein providing a biocompatible small molecule crosslinker further comprises providing a biocompatible small molecule crosslinker having a biodegradable link.

10. The composition of claim 1, wherein providing a synthetic biocompatible functional polymer further comprises providing a synthetic biocompatible functional polymer having a biodegradable link.

11. The composition of claim 1, wherein permitting the crosslinker and functional polymer to react further comprises reacting the crosslinker functional groups and the functional polymer functional groups to produce a biodegradable link.

12. The composition of claim 1, wherein the hydrogel further comprises a dye.

13. The composition of claim 1, wherein the hydrogel further comprises one or more active ingredients.

14. The composition of claim 13, wherein the active ingredient comprises enzymes, vasoconstrictors, chemotherapeutic agents, antimicrobials, antibiotics, and combinations thereof.

15. The composition of claim 1, wherein the hydrogel forms over a period of time of from about 5 seconds to about 90 seconds.

## Patentansprüche

1. Zusammensetzung, die ein biokompatibles kleines Vernetzermolekül mit einer Molekülmasse von 2000 oder weniger umfasst, wobei der Vernetzer n funktionelle Vernetzergruppen aufweist, wobei n für zwei oder mehr steht, und wobei die funktionellen Vernetzergruppen entweder elektrophil oder nukleophil sind, in Kombination mit einem synthetischen biokompatiblen funktionellen Polymer mit einer Molekülmasse von mindestens dem ungefähr 7-fachen von dem des Vernetzers, wobei das funktionelle Polymer m funktionelle Polymer-funktionelle Gruppen umfasst, wobei m für zwei oder mehr steht und die Summe von n und m gleich fünf oder größer ist, und wobei die funktionellen Polymer-funktionellen Gruppen nukleophil sind, wenn die funktionellen Vernetzergruppen elektrophil sind, und die funktionellen Polymer-funktionellen Gruppen elektrophil sind, wenn die funktionellen Vernetzergruppen nukleophil sind, zur Verwendung in einem Verfahren, wobei das Verfahren das Einführen der Zusammensetzung in Kombination mit dem synthetischen biokompatiblen Polymer in ein Gewebe, welches die Submukosa eines Polypen umfasst, und das Gestatten der Umsetzung des Vernetzers und funktionellen Polymers in der Submukosa des Polypen, um ein Hydrogel zu bilden; und Entfernen des Polypen umfasst.

2. Zusammensetzung nach Anspruch 1, wobei das Bereitstellen eines biokompatiblen kleinen Vernetzermoleküls ferner das Bereitstellen eines biokompatiblen kleinen Vernetzermoleküls umfasst, das eine Löslichkeit von mindestens 1 g/100 mL in einer wässrigen Lösung aufweist.

3. Zusammensetzung nach Anspruch 1, wobei das Bereitstellen eines biokompatiblen kleinen Vernetzermoleküls ferner das Bereitstellen eines biokompatiblen kleinen Vernetzermoleküls umfasst, das funktionelle Vernetzergruppen aufweist, die elektrophil sind.

4. Zusammensetzung nach Anspruch 3, wobei das Bereitstellen eines biokompatiblen kleinen Vernetzermoleküls, das funktionelle Vernetzergruppen aufweist, die elektrophil sind, ferner das Bereitstellen eines biokompatiblen kleinen Vernetzermoleküls umfasst, wobei die funktionellen elektrophilen Vernetzergruppen Vernetzergruppen auf der Basis von N-Hydroxysuccinimid sind.

5. Zusammensetzung nach Anspruch 3, wobei das Bereitstellen eines synthetischen biokompatiblen funktionellen Polymers ferner das Bereitstellen eines synthetischen biokompatiblen funktionellen Polymers umfasst, wobei die funktionellen Polymer-funktionellen Gruppen Amine sind.

6. Zusammensetzung nach Anspruch 1, wobei das Bereitstellen eines biokompatiblen kleinen Vernetzermoleküls ferner das Bereitstellen eines biokompatiblen kleinen Vernetzermoleküls umfasst, das funktionelle Vernetzergruppen aufweist, die nukleophil sind.

7. Zusammensetzung nach Anspruch 6, wobei das Bereitstellen eines biokompatiblen kleinen Vernetzermoleküls, das funktionelle Vernetzergruppen aufweist, die nukleophil sind, ferner das Bereitstellen eines biokompatiblen kleinen Vernetzermoleküls umfasst, wobei die funktionellen Vernetzergruppen Amine sind.

8. Zusammensetzung nach Anspruch 6, wobei das Bereitstellen eines synthetischen biokompatiblen funktionellen Polymers ferner das Bereitstellen eines synthetischen biokompatiblen funktionellen Polymers umfasst, wobei die funktionellen Polymer-funktionellen Gruppen N-Hydroxysuccinimid-Gruppen sind.

9. Zusammensetzung nach Anspruch 1, wobei das Bereitstellen eines biokompatiblen kleinen Vernetzermoleküls ferner das Bereitstellen eines biokompatiblen kleinen Vernetzermoleküls umfasst, das eine biologisch abbaubare Verknüpfung aufweist.

10. Zusammensetzung nach Anspruch 1, wobei das Bereitstellen eines biokompatiblen synthetischen funktionellen Polymers ferner das Bereitstellen eines biokompatiblen synthetischen funktionellen Polymers umfasst, das eine biologisch abbaubare Verknüpfung aufweist.

11. Zusammensetzung nach Anspruch 1, wobei das Gestatten der Umsetzung des Vernetzungsmittels und des funktionellen Polymers ferner das Umsetzen der funktionellen Vernetzergruppen und der funktionellen Polymer-funktionellen Gruppen umfasst, um eine biologisch abbaubare Verknüpfung herzustellen.

12. Zusammensetzung nach Anspruch 1, wobei das Hydrogel ferner einen Farbstoff aufweist.

13. Zusammensetzung nach Anspruch 1, wobei das Hydrogel ferner einen oder mehrere Wirkstoffe aufweist.

14. Zusammensetzung nach Anspruch 13, wobei der Wirkstoff Enzyme, Vasokonstriktoren, chemotherapeutische Mittel, antimikrobielle Mittel, Antibiotika und Kombinationen davon umfasst.

15. Zusammensetzung nach Anspruch 1, wobei das Hydrogel sich über einen Zeitraum von ungefähr 5 Sekunden bis ungefähr 90 Sekunden bildet.

## Revendications

1. Composition comprenant un agent de réticulation à petite molécule compatible d'un point de vue biologique ayant une masse moléculaire de 2 000 ou moins, l'agent de réticulation ayant n groupes fonctionnels d'agent de réticulation, dans laquelle n vaut deux ou plus, et dans laquelle les groupes fonctionnels d'agent de réticulation sont soit électrophiles soit nucléophiles, en combinaison avec un polymère fonctionnel synthétique compatible d'un point de vue biologique avec une masse moléculaire d'au moins environ 7 fois plus que l'agent de réticulation, le polymère fonctionnel ayant m groupes fonctionnels de polymère fonctionnel, dans laquelle m vaut deux ou plus et la somme de n et m est de cinq ou plus, et dans laquelle les groupes fonctionnels de polymère fonctionnel sont nucléophiles si les groupes fonctionnels d'agent de réticulation sont électrophiles, et les groupes fonctionnels de polymère fonctionnel sont électrophiles si les groupes fonctionnels d'agent de réticulation sont nucléophiles pour une utilisation dans un procédé dans lequel le procédé comprend l'introduction dans un tissu comprenant une sous-muqueuse d'un polype de ladite composition en combinaison avec ledit polymère fonctionnel synthétique compatible d'un point de vue biologique et la permission accordée à l'agent de réticulation et au polymère fonctionnel de réagir dans la sous-muqueuse du polype pour former un hydrogel ; et
l'enlèvement du polype.

2. Composition selon la revendication 1, dans laquelle la fourniture d'un agent de réticulation à petite molécule compatible d'un point de vue biologique comprend en outre la fourniture d'un agent de réticulation à petite molécule compatible d'un point de vue biologique ayant une solubilité d'au moins 1 g/100 ml dans une solution aqueuse.

3. Composition selon la revendication 1, dans laquelle la fourniture d'un agent de réticulation à petite molécule compatible d'un point de vue biologique comprend en outre la fourniture d'un agent de réticulation à petite molécule compatible d'un point de vue biologique ayant des groupes fonctionnels d'agent de réticulation qui sont électrophiles.

4. Composition selon la revendication 3, dans laquelle la fourniture d'un agent de réticulation à petite molécule compatible d'un point de vue biologique ayant des groupes fonctionnels d'agent de réticulation qui sont électrophiles comprend en outre la fourniture d'un agent de réticulation à petite molécule compatible d'un point de vue biologique dans lequel les groupes fonctionnels d'agent de réticulation électrophiles sont des groupes d'agent de réticulation à base de N-hydroxysuccinimide.

5. Composition selon la revendication 3, dans laquelle la fourniture d'un polymère fonctionnel synthétique compatible d'un point de vue biologique comprend en outre la fourniture d'un polymère fonctionnel synthétique compatible d'un point de vue biologique dans lequel les groupes fonctionnels de polymère fonctionnel sont des amines.

6. Composition selon la revendication 1, dans laquelle la fourniture d'un agent de réticulation à petite molécule compatible d'un point de vue biologique comprend en outre la fourniture d'un agent de réticulation à petite molécule compatible d'un point de vue biologique ayant des groupes fonctionnels d'agent de réticulation qui sont nucléophiles.

7. Composition selon la revendication 6, dans laquelle la fourniture d'un agent de réticulation à petite molécule compatible d'un point de vue biologique ayant des groupes fonctionnels d'agent de réticulation qui sont nucléophiles comprend en outre la fourniture d'un agent de réticulation à petite molécule compatible d'un point de vue biologique dans lequel les groupes fonctionnels d'agent de réticulation sont des amines.

8. Composition selon la revendication 6, dans lequel la fourniture d'un polymère fonctionnel synthétique compatible d'un point de vue biologique comprend en outre la fourniture d'un polymère fonctionnel synthétique compatible d'un point de vue biologique dans lequel les groupes fonctionnels de polymère fonctionnel sont des groupes N-hydroxysuccinimide.

9. Composition selon la revendication 1, dans laquelle la fourniture d'un agent de réticulation à petite molécule compatible d'un point de vue biologique comprend en outre la fourniture d'un agent de réticulation à petite molécule compatible d'un point de vue biologique ayant une liaison biodégradable.

10. Composition selon la revendication 1, dans laquelle la fourniture d'un polymère fonctionnel synthétique compatible d'un point de vue biologique comprend en outre la fourniture d'un polymère fonctionnel synthétique compatible d'un point de vue biologique ayant une liaison biodégradable.

11. Composition selon la revendication 1, dans laquelle la permission accordée à l'agent de réticulation et au polymère fonctionnel de réagir comprend en outre la réaction des groupes fonctionnels d'agent de réticulation et des groupes fonctionnels de polymère fonctionnel pour produire une liaison biodégradable.

12. Composition selon la revendication 1, dans laquelle l'hydrogel comprend en outre un colorant.

13. Composition selon la revendication 1, dans laquelle l'hydrogel comprend en outre un ou plusieurs ingrédients actifs.

14. Composition selon la revendication 13, dans laquelle l'ingrédient actif comprend des enzymes, des vasoconstricteurs, des agents chimiothérapeutiques, des produits antimicrobiens, des antibiotiques et des combinaisons de ceux-ci.

15. Composition selon la revendication 1, dans laquelle l'hydrogel se forme sur une période de temps d'environ 5 secondes à environ 90 secondes.
